# EUROPEAN PATENT SPECIFICATION

(11) **EP 3 429 666 B1**
(45) Date of publication and mention of the grant of the patent: **23.02.2022**
(21) Application number: 17712012.8
(22) Date of filing: 13.03.2017
(51) Int. Cl.: A61M 5/315

(54) **AN INJECTOR WITH REDUCED BREAK LOOSE FORCE**
SELBSTKOMPENSIERENDER KOLBEN
PISTON AUTO-COMPENSATEUR

(30) Priority: 13.03.2016 DK 201600154; 03.04.2016 DK 201600198; 08.08.2016 DK 201670589
(43) Date of publication of application: 23.01.2019
(73) Proprietor: Injecto Group A/S, 2900 Hellerup (DK)
(72) Inventor: HETTING, Mikael, 2920 Charlottenlund (DK)
(74) Representative: AWA Denmark A/S
(86) International application number: PCT/DK2017/050070
(87) International publication number: WO 2017/157396

(56) References cited:
- EP-A1- 1 264 611
- EP-A1- 2 218 473
- DE-A1- 4 428 467
- JP-A- 2000 070 367
- US-A1- 2003 105 433
- US-A1- 2015 119 817

## Description

### Field of the invention

The present invention relates to injector having a reduced break loose force (BLF). The injector comprises a cylinder extending along a longitudinal axis, the cylinder having an inner wall, an outer wall, and an outlet at an outlet end opposite an actuating end, and a piston assembly. The injector of the invention provides a reduced BLF, and is especially suited for pre-filled injectors allowing long term storage of pharmaceutical compositions.

### Prior art

An injector for delivery of a pharmaceutical composition typically comprises a piston in a cylinder so that the piston can be pushed from one end of the cylinder to the other thereby ejecting liquid contained in the cylinder. A piston in a cylinder will abut the inner wall of the cylinder and at the interface between the piston and the inner wall there will be a static friction and a dynamic friction. Movement of the piston in the cylinder will require application of a force sufficient to overcome initially the static friction and subsequently the dynamic friction; the static friction will be larger than the dynamic friction and thereby the force to provide an initial movement of the piston is larger than the force required to provide a sustained movement of the piston. Once the piston has stopped moving the force to provide an initial movement must be overcome again. Traditionally, the inner wall of the cylinder is lubricated in order to keep the dynamic friction sufficiently low to ensure sufficient glide for the piston and allow for easy movement of the piston in the cylinder and thereby easy delivery of a pharmaceutical composition during injection. The static friction is often, in the context of pharmaceutical injectors, referred to as the "break loose force" (BLF), and the dynamic friction is commonly referred to as the "glide force". In order to provide injectors that are pleasant and easy to use there is an interest in providing injectors having low BLF values.

BLF depends on several factors but is especially sensitive to increased duration of the interaction between the piston's sealing elements and the inner wall of the syringe. Unlike traditional syringes which are filled and emptied within a few minutes prefilled syringes are shelved over longer periods of time which causes a tendency for the sealing elements to attach themselves to the inner wall of the syringe, which is also known as the "stick effect" and which increases over time and is further increased by ambient adverse conditions such as increased temperature, humidity, etc.

BLF is especially critical concerning prefilled syringes since these are shelved in periods of up to three years. Normally the problem concerning high BLF is solved by lubricating the piston and/or the inner wall of the syringe, but tests have shown that silicone lubrication may often have an adverse effect causing proteins in liquid injectable pharmaceutical compositions to aggregate leaving them inefficient or even harmful to the patient receiving the injection. Moreover, in connection with ophthalmic injections the silicone lubricant has a tendency to remain in the eye, which alone is undesirable. BLF is even more critical concerning glass syringes, especially prefilled syringes, since the barrels of these unlike precision moulded plastic barrels are manufactured with substantially higher tolerances leading to pistons which either fit too tightly or the opposite, dictating the use of lubrication. For the inner diameter of a glass syringe the container inner diameter may vary up to plus or minus 1/10 millimetre resulting in a total discrepancy of up to 0.2 mm from one container to another which has a significant influence on the friction between piston and the container inner wall and hence the BLF, and since the sealing is absolutely imperative in order to comply with the standards of container closure integrity (CCI) existing pistons for glass barrels must have an outer diameter which is significantly larger than the corresponding diameter when the barrel diameter is on the low side.

Several suggestions exist in the prior art to address various problems relating to the general user comfort of syringes or to achieve other more specific aims.

US 2003/105433 discloses a disposable syringe with a pneumatic chamber formed between a master plunger and a slave plunger. In use, pressure is applied to the master plunger to slide it towards the slave plunger, and since the gas in the pneumatic chamber is more compressible than the liquid in the liquid chamber, the slave plunger will move when there is enough pressure to cause the slave plunger to begin to slide and push the liquid out of the needle. Compressing the air in the pneumatic chamber provides an "air cushion", which allows for a more controlled injection of the liquid, since slight variations in the pressure asserted by the user of the syringe will be absorbed by the compressing/decompressing forces acting in concert with the air cushion.

EP 2218473 addresses the same problem as US 2003/105433 with respect to dampening pressure variations in dental injections and being able to aspirate liquid in the syringe during an injection is considered important. The syringe of EP 2218473 also employs an air cushion between to plunger parts, and it may comprise a pressure indicator in the gas volume.

DE 4428467 discloses a syringe with an injection piston where a dampening effect is relevant for spinal anaesthesia and intravenous injections. The syringe has an injection piston, a compression piston and a compressible medium between the two piston elements. The syringe may further comprise a spring but no effect is ascribed to the spring.

US 2006/247582 discloses a "flush syringe" for use in flush procedures for vascular access devices. The flush syringe is designed to ensure that reflux does not occur following a flush procedure, and it has a stopper with a distal and a proximal stopper portion separated by a spring means for moving the distal stopper portion in a distal direction to drive more liquid out of the chamber of the syringe after engaging the stopper. Evidently the flush syringe is not concerned with lowering the BLF.

Several manufacturers within the industry have sought to eliminate the addition of silicone lubricants and instead other solutions have been introduced with various baked-on surfaces or the like, which are supposed to reduce the force needed to initiate movement of the piston. Numerous suggestions exist in the prior art to address the problem of lowering the BLF and these typically involve modifications of the lubricant, for example, how the lubricant is applied or the type of lubricant, e.g. with respect to viscosity. As an alternative WO 2014/194918 suggests how the BLF may be controlled using special designs of the piston.

It is expected that further improvements are possible and it is an aim of the present invention to provide an injector having a reduced BLF. The invention is mainly dedicated to prefilled syringes although it aims to be of utility for other medical applications and purposes.

### Disclosure of the invention

The present invention relates to an injector, which injector does not comprise a silicone lubricant and is suitable for delivery of a pharmaceutical composition. The injector comprises
- a cylinder extending along a longitudinal axis, the cylinder having an inner wall, an outer wall, and an outlet at an outlet end opposite an actuating end,
- a piston assembly comprising a compressible section between an actuating piston element having a Shore A hardness in the range of 50 to 90 and a passive piston element having a Shore A hardness in the range of 50 to 90, which piston elements abut the inner wall of the cylinder at abutting interfaces thereby sealing the compressible section when the piston assembly is inserted in the cylinder, the compressible section comprising a compressible fluid and a resilient frame, the resilient frame physically coupling the actuating piston element to the passive piston element, which resilient frame is made from an elastic polymer and has a volumetric fraction in the range of 15% to 50% of the volume of the compressible section and which resilient frame has an axial length in the range of 25% to 90% of the total axial length of the piston assembly in a relaxed state, and in that the compressible fluid is entrapped between the actuating piston element and the passive piston element.

The compressible section comprises a compressible fluid and a resilient frame linking the actuating piston element to the passive piston element. In the context of the invention the term "linking" means that the actuating piston element and the passive piston element are physically coupled to each other so that actuating one of the elements will also actuate the other element. For example, if the actuating piston element is pushed the resilient frame will push the passive piston element, and if the actuating piston element is pulled the resilient frame will pull the passive piston element. By including a compressible section between an actuating piston element and a passive piston element in a piston assembly the break loose force (BLF) of the piston assembly can be lowered compared to a piston assembly where the actuating piston element and the passive piston element are rigidly coupled to each other. Thus for example, a piston assembly having an actuating piston element and a passive piston element with an air cushion between them will have a reduced BLF. However, the present inventor has now surprisingly found that by linking the actuating piston element to the passive piston element with the resilient frame according to the invention the overall BLF may be aligned with and have a value similar to the glide force of the piston assembly. This provides an even smoother experience to the end user of the piston, e.g. than can be obtained using an air cushion, since the BLF will not even be recognised during operation of the syringe by being of similar to the glide force.

The injector comprises a cylinder. In the context of the invention a "cylinder" is any kind of tube or the like allowing the piston assembly to be moved from one position in the cylinder to another. The cylinder has an "actuating end" and "outlet end" opposite each other. The actuating end of the cylinder allows access to the piston assembly for moving it, i.e. "actuating" the piston assembly via the actuating piston element, in the cylinder. The outlet end of the cylinder comprises an outlet for a fluid contained in the cylinder.

The cylinder has an inner wall and an outer wall. The inner wall is generally smooth without any protrusions, indentations or the like. In particular, the piston assembly may preferably provide a seal throughout the axial length of the cylinder. The outer wall may comprise a finger grip positioned anywhere along the length of the cylinder, e.g. at the actuating end of the cylinder. In an embodiment the cylinder comprises a finger grip positioned between the outlet end and the actuating end together with a needle guard for mounting on the outside of the cylinder as described in WO 2016/192739, to which reference is herewith made, in particular p. 3, lin. 7 to p. 5, lin. 14. The needle guard is described on p. 6, lin. 3 to 7, lin. 25 of WO 2016/192739 and the corresponding figures. Likewise, the text of claims 1 to 9 of WO 2016/192739 is also referred to.

The cylinder may be made from any relevant material, and typical materials comprise polymeric materials, such as cyclic olefin copolymer (COC), e.g. TOPAS polymers (supplied by TOPAS Advanced Polymers GmbH), cyclic olefin polymer (COP), e.g. Zeonor, or polystyrene, or glasses, e.g. borosilicate glasses. COC polymers are advantageous due to their excellent barrier characteristics and thus accommodate the need for long-term storage of pharmaceutical agents. In another embodiment the cylinder is made from glass, e.g. borosilicate glass. It is also contemplated that the cylinder may be made from a metal or it may comprise any combination of polymeric materials, glasses or metals. The cross-sectional shape of the cylinder is not limited although it is preferred that the cylinder has a round cross-section. It is also contemplated that the cross-section may be oval, elliptical, polygonal, etc. When the cylinder has a round cross-section the diameter, e.g. the inner diameter, may have any value conventionally used with syringes. For example, in a preferred embodiment the cylinder has an inner diameter in the range of 2 mm to 10 mm, such as 4.65 mm, 6.35 mm or 8.80 mm, although it may have larger values according to the invention.

The piston assembly is especially suitable for lubrication free interaction with glass syringe barrels which have inner diameter tolerances too wide to interact with traditional pistons and omit lubrication at the same time due to too high BLF values for the traditional piston. At low diameter values the BLF will be too high and at high diameter values the container closure integrity (CCI) will be compromised with traditional pistons. In contrast the piston assembly employed in the present invention is capable of compensating for the wide tolerances due to its displaced movement of the sealing elements which allows for a larger piston diameter while still keeping the BLF within acceptable values, at the same time enabling omittance of traditional lubrication.

The injector of the invention has a piston assembly with an actuating piston element and a passive piston element; the actuating piston element is the piston element closer to the actuating end of the cylinder and the passive piston element is the piston element closer to the outlet end of the cylinder. The actuating piston element and the passive piston element may be identical, or the actuating piston element may be different compared to the passive piston element. The actuating piston element has an actuating surface facing the actuating end of the cylinder and the passive piston element has an outlet surface opposite the actuating surface thus facing the outlet end of the cylinder. Each piston element may have an inner surface facing the compressible section.

The piston assembly has a total length calculated from the actuating surface of the actuating piston element to the outlet surface of the passive piston element, i.e. when the piston assembly is in a relaxed state and not subjected to external pressure from an end user. The axial length of the compressible section is in the range of 25% to 90% of the total axial length of the piston assembly in a relaxed state. The actuating piston element and the passive piston element generally have low compressibility, e.g. piston bodies are not compressible. In a specific embodiment the axial length of the compressible section is at least 30% of the total axial length of the piston assembly in a relaxed state. Thus, the resilient frame may have a length of 30% to 70%, e.g. such as 30%, 40%, 50%, 60% or 70%, of the total axial length of the piston assembly in a relaxed state.

The piston elements provide liquid tight seal, and optionally also an airtight seal, between the respective piston elements and the inner wall of the cylinder, thereby forming the compressible section between the actuating piston element and the passive piston element. Thus, when the actuating piston element is pushed from the actuating end of the cylinder the compressible section will be compressed and eventually push the passive piston element towards the outlet end of the cylinder thereby ejecting liquid contained in the cylinder. The actuating piston element and the passive piston element each have a static friction, which must be overcome before the respective piston starts moving in the cylinder. In contradiction to existing pistons the piston according to the invention reduces the BLF to move the piston to significantly lower values by introducing displaced movement of the sealing elements equally dividing the total BLF into smaller increments by stepwise movement of the sealing elements. When the actuating piston has started moving its movement will push the passive piston element, but the present inventor has now surprisingly found that the static friction of the passive piston element only contributes insignificantly to the force needed to move the piston assembly and eject liquid from the cylinder so that a smooth movement of the piston assembly is obtained with the static friction of only the actuating piston element contributing to the static friction of the piston assembly. The resilient frame further contributes to the effect on the BLF so that when a resilient frame is present in the piston assembly, e.g. a resilient frame having an axial length of at least 25% of the total length of the piston assembly, the BLF (measured in N) is less than twice as high as the glide force (measured in N), e.g. the BLF and the glide force are approximately equal, thus providing an even smoother experience to the end user.

By having a piston assembly with two piston elements a more efficient sealing of the contents in the cylinder, e.g. a pharmaceutical composition, such as a vaccine, is obtained than by using a piston with a single piston element. This is particularly relevant for a syringe prefilled with a pharmaceutical composition. If a traditional piston, i.e. without the compressible section, were to employ two sealing elements corresponding to the piston elements of the piston assembly of the invention a much higher BLF would be observed making use of the injector less convenient. Thus, the invention provides a user friendly injector with efficient sealing of a pharmaceutical composition in the injector. The present inventor believes, without being bound by any particular theory, that the resilient nature of the compressible section provides an evening-out of the force applied to the passive piston element, which in turn lowers the total BLF of the piston assembly. In further embodiments the injector may comprise more than two piston elements with a compressible section between pairs of piston elements.

The maximum BLF felt by the user will thus never be more than the BLF for one piston element. The following values exemplify the BLF force obtained in an embodiment of the invention: the BLF of the actuating piston element of about 7 N reduced to a mean glide force of about 4 N increased to a BLF of the passive piston element of about 7 N reduced to a mean glide force of about 4 N for injection completion, equivalent to approximately 50% of the BLF of a traditional piston of about 14 N. The piston according to the invention is hence able to reduce the BLF to half value or lower compared to any known piston, and moreover when the resilient frame is present the glide force is typically more than half the BLF thereby providing an even smoother end user experience.

The piston assembly comprises the actuating piston element and the passive piston element, and a compressible section comprising a compressible fluid. Any non-compressible fluid present between the actuating piston element and the passive piston element is considered not to be part of the compressible section. For example, a compressible fluid, e.g. air, between the actuating piston element and the passive piston element will normally contain humidity, which can form droplets of water on the inner wall of the cylinder or the inner surfaces of the piston elements. Once the actuating piston element is moved the compressible fluid will be compressed, and eventually the pressure in the compressible section will be large enough to overcome the static friction of the passive piston element, which will start moving. The compressible fluid will provide a spring-like effect so that the end user will have a smoother experience than if he were to operate an injector having a single stopper with two piston elements, where both sealing elements will have to initiate movement simultaneously resulting in a significantly higher BLF value.

The compressible section comprises a compressible fluid and a resilient frame linking the actuating piston element to the passive piston element. The resilient frame may take any form desired, and it may be prepared from any material providing appropriate resilience. The material between the actuating piston element and the passive piston element may, in the context of this invention, also be referred to as a "stem" or "stems". For example, material may be removed from a block of material to leave stems, which make up the frame. The resilient frame may for example be made from an elastic polymer, e.g. a thermoplastic elastomer (TPE), or from an elastic metal. In a certain embodiment the resilient frame is injection moulded from a TPE, e.g. a styrene block copolymer (SBC), such as SBC selected from the list consisting of hydrogenated SBC or non-hydrogenated SBS or alloys of these.

Likewise, the actuating piston element and the passive piston element may also be prepared from any appropriate material as desired. However, the actuating piston element and the passive piston element will typically be prepared from a polymeric material, in particular an elastic polymeric material allowing that the respective piston element seals between the piston element and the inner wall of the cylinder. Preferred materials for the actuating piston element and the passive piston element, and also the resilient frame, are TPE's, e.g. an SBC, such as an SBC selected from the list consisting of hydrogenated SBC or non-hydrogenated SBS or alloys of these. It is further preferred that the actuating piston element and the passive piston element are injection moulded from a TPE, e.g. the TPE's mentioned above. Thus, in an embodiment the actuating piston element, the passive piston element, the resilient frame, or the actuating piston element, the passive piston element and the resilient frame, e.g. as a single piece, are made from a TPE.

In an embodiment, the actuating piston element, the passive piston element, the resilient frame, or the actuating piston element, the passive piston element and the resilient frame, e.g. as a single piece, are made by injection moulding.

Where a TPE is appropriate in the present invention, any TPE may be used. Appropriate thermoplastic polymers comprise SBCs, e.g. hydrogenated - H-SBC - (SEBS - styreneethylene butylenes-styrene or similar) or non-hydrogenated (SBS - styrene-butadienestyrene) or alloys of these and other compatible polymers, such as COC elastomers. Preferred SBCs are those known under the trademark Evoprene as marketed by AlphaGary Corporation (Leominster, MA, USA). Evoprenes are described in the brochure "EVOPRENE^{™} Thermoplastic Elastomer (TPE) Compounds - GENERAL INFORMATION" (published by AlphaGary, July 2007), and preferred Evoprene^{™} polymers are Evoprene^{™} Super G, Evoprene^{™} G, Evoprene^{™} GC, and Evoprene^{™} HP, which are described in the brochures "EVOPRENE^{™} SUPER G Thermoplastic Elastomer (TPE) Compounds", "EVOPRENE^{™} G Thermoplastic Elastomer (TPE) Compounds", "EVOPRENE^{™} GC Thermoplastic Elastomer (TPE) Compounds", and EVOPRENE^{™} HP Thermoplastic Elastomer (TPE) Compounds (published by AlphaGary, July 2007), respectively. Other relevant elastomers comprise COC elastomers, e.g. TOPAS^{®} Elastomer E-140.

When the piston assembly is injection moulded the piston assembly can be made with lower tolerances than afforded by technologies such as vulcanisation, which is commonly used in the manufacture of traditional rubber pistons, e.g. pistons made from bromobutyl or chlorobutyl rubber. Appropriate materials comprise elastomers, such as rubbers, e.g. natural rubber, synthetic rubber (polyisoprene rubber, butyl rubber), silicone rubber, and the like, which may be defined with respect to e.g. the Shore durometer, which indicates the elasticity of the elastomeric material and measures the hardness of the elastomeric material, where the higher the durometer, the harder the compound. For example, in an embodiment of the invention the piston assembly, e.g. including a deformable sealing element, has a Shore A hardness in the range of 50 to 90, preferably 60 to 80, more preferred 71 to 76. The terms "Shore hardness" and "Shore durometer" may be used interchangeably. In general, the deformable sealing element will be homogeneous and composed of the same material throughout the volume of the deformable sealing element, which material has a Shore A hardness in the given ranges. By using a material with a Shore A hardness in the above mentioned range, a relatively hard elastomeric material is provided. It should be noted that Shore A durometer is only one of many ways to characterise the material properties of the chosen material, and that other tests may also be employed to characterise the material. Measurement of the Shore A hardness is well-known to the skilled person and in particular the Shore A hardness is generally recorded according to the ISO 868 standard.

Exemplary TPE's and their Shore A hardnesses are summarised in Table 1.

**Table 1 Exemplary TPE's**

| | | | | | |
|---|---|---|---|---|---|
| Evoprene G 3295 | 57 | Evoprene GC 5648 | 55 | Evoprene GC 5700 | 75 |
| Evoprene G 6087 | 86 | Evoprene GC 5649 | 63 | Evoprene GC 5701 | 86 |
| Evoprene G 614 | 88 | Evoprene GC 5650 | 71 | Evoprene GC 640 | 80 |
| Evoprene G 927 | 82 | Evoprene GC 5651 | 76 | Evoprene GC 641 | 76 |
| Evoprene G 940 | 87 | Evoprene GC 5652 | 87 | Evoprene GC 642 | 76 |
| Evoprene G 958 | 56 | Evoprene GC 5656 | 54 | Evoprene GC 645 | 86 |
| Evoprene G 962 | 50 | Evoprene GC 5657 | 60 | Evoprene HP 3704 | 50 |
| Evoprene G 963 | 60 | Evoprene GC 5658 | 69 | Evoprene HP 3706 | 60 |
| Evoprene G 964 | 88 | Evoprene GC 5659 | 76 | Evoprene HP 3708 | 70 |
| Evoprene G 969 | 65 | Evoprene GC 5681 | 59 | Evoprene HP 3710 | 80 |
| Evoprene G 970 | 70 | Evoprene GC 5682 | 67 | Evoprene HP 3712 | 90 |
| Evoprene G 971 | 83 | Evoprene GC 5683 | 76 | Evoprene HP 3724 | 50 |
| Evoprene G 974 | 71 | Evoprene GC 5684 | 85 | Evoprene HP 3726 | 60 |
| Evoprene G 975 | 84 | Evoprene GC 5688 | 53 | Evoprene HP 3728 | 70 |
| Evoprene G 978 | 91 | Evoprene GC 5689 | 61 | Evoprene HP 3730 | 80 |
| Evoprene G 991 | 60 | Evoprene GC 5690 | 67 | Evoprene HP 3732 | 90 |
| Evoprene G 996 | 74 | Evoprene GC 5691 | 78 | Evoprene Super G 931 | 61 |
| Evoprene GC 5617 | 59 | Evoprene GC 5692 | 83 | Evoprene Super G 932 | 71 |
| Evoprene GC 5618 | 65 | Evoprene GC 5693 | 86 | Evoprene Super G 934 | 76 |
| Evoprene GC 5619 | 73 | Evoprene GC 5698 | 52 | Evoprene Super G 949 | 54 |
| Evoprene GC 5647 | 50 | Evoprene GC 5699 | 63 | TOPAS^{®} E-140 | 89 |

In the piston assembly according to the invention, the passive piston element, and the resilient frame have, or the actuating piston element, the passive piston element and the resilient frame may have a Shore A hardness in the range of 50 to 90. When the resilient frame has a Shore A hardness in the range of 50 to 90, in particular 70 to 90, the BLF (measured in N) is generally within 150% of the glide force (measured in N).

TPE's may also be defined by their compression set values, which corresponds to the deformation remaining after removal of a force that was applied to it (and is typically expressed in %). The compression set value is typically recorded over a specified period of time, e.g. in the range of 18 hours to 96 hours, and at a specified temperature, for example according to the ISO 815 standard. In the context of the present invention the compression set is generally recorded at an "ambient temperature", e.g. in the range of 10°C to 40°C. In general, the higher the temperature the shorter the time relevant for recording the compression set. The compression set should generally be as low as possible but for a piston assembly, or a part of a piston assembly, of the invention the compression set may be in the range of 15% to 40%. The compression set value is generally relevant for prefilled injectors where the piston assembly will be inserted into the cylinder and therefore compressed when the prefilled injector is stored for extended periods of time. When the piston elements have a Shore A hardness in the range of 50 to 90 and a compression set value of at least 25%, e.g. in the range of 25% to 35%, the BLF of a prefilled injector of the invention will decrease upon storage, e.g. for at least 5 days, so that a piston assembly of the invention is especially advantageous for a prefilled injector. As long as the compression set is below 60% CCI is ensured.

In a certain embodiment the piston elements of the piston assembly each comprise one or more convex deformable sealing elements, which deformable sealing element abuts the inner wall of the cylinder at an abutting interface and provide a seal between the piston element and the inner wall of the cylinder. The term "abutting interface" refers to any section where the inner wall and the deformable sealing elements contact each other and the "abutting interface" does not impose any limitations on either the inner wall of the cylinder or the surface of the sealing element. In this context the term "convex" means that a straight line between any two points within the deformable sealing element does not cross the surface of the deformable sealing element. In particular, the deformable sealing elements are convex when the respective piston element is in a "relaxed state", e.g. in a state without deformation, such as deformation caused by inserting the piston assembly into a cylinder, although it is preferred that the deformable sealing elements are also convex when inserted into the cylinder. The diameter of the deformable sealing element, i.e. the diameter of the piston element, is typically 3% to 20% larger than the inner diameter of the cylinder, e.g. 5% to 15% larger. When a piston element with a convex deformable sealing element having a diameter 3% to 20% larger than the inner diameter of the cylinder is prepared from a material, e.g. a TPE, of a Shore A hardness in the range of 50 to 90, e.g. in the range of 70 to 90, the piston assembly may be used without lubrication. The injector does not comprise a silicone lubricant. Lubricants, e.g. silicone lubricants, are not inert with respect to certain pharmaceutical compounds, e.g. pharmaceutical compounds based on protein molecules, such as vaccines, and lubricants should be avoided for long term storage of injectors prefilled with a pharmaceutical compound. Therefore, this advantageously allows long term storage of an injector of the invention prefilled with a pharmaceutical compound, e.g. a protein based pharmaceutical compound, without detrimental effects on the pharmaceutical compound. When the actuating piston element and the passive piston element, in particular the deformable sealing elements that contact the inner surface of the cylinder, are made from a TPE having a Shore A hardness in the range of 50 to 90, the stick-in effect is avoided even when no lubricant, e.g. a silicone lubricant, is present. Thus, any embodiment where the actuating piston element and the passive piston element are made from a TPE having a Shore A hardness in the range of 50 to 90 and where no lubricant is present is especially suited for long term storage, since no stick-in effect occurs, long term sealing is provided and negative effects on the pharmaceutical from a lubricant are avoided while still retaining a smooth end user experience due to the reduction in the BLF afforded by the piston assembly of the invention. The effect is observed for injectors having polymeric as well as glass cylinders.

In a specific embodiment the diameter of the actuating piston element is smaller than the diameter of the passive piston element, e.g. when the piston assembly is in a relaxed state. Thereby, the BLF relating to the actuating piston element is smaller than the BLF relating to the passive piston element, which in turn provides that both the overall BLF and the glide force is decreased thus providing a smoother experience to the end user when emptying the injector. Moreover, since the BLF of the actuating piston element is lower than the BLF of the passive piston element it is ensured that when the actuating force is removed from the actuating piston element the passive piston element will remain and only the actuating piston element will move. Thereby better control of the injected volume is possible.

Due to its unique design reducing the total BLF by differential movement of sealing elements the piston assembly is able to interact smoothly with glass barrels despite the significant tolerances even when lubrication is omitted, since the diameters of the piston elements may be maximised while still obtaining a low BLF due to the differential movement of the piston elements. Thus, in a preferred embodiment the cylinder is made from glass, e.g. borosilicate glass, and the injector does not comprise a lubricant. The combination of the efficient sealing obtained using two piston elements and the reduced BLF is especially advantageous for long term storage of a prefilled injector, since the stick-in effect does not occur, and moreover detrimental effects of lubricants on the pharmaceutical composition, e.g. a protein based pharmaceutical composition, are avoided. The invention can be said to provide an injector for long term storage of a pharmaceutical composition, which does not suffer from stick-in effects.

In an embodiment the resilient frame is joined with the actuating piston element and the passive piston element by gluing, welding, e.g. laser welding, ultrasonic welding, etc., or by any appropriate method. In this embodiment the actuating piston element, the passive piston element and the resilient frame may be made from the same material or from different materials.

In another embodiment the actuating piston element, the passive piston element and the resilient frame are provided as a single piece of material, which may be modified e.g. to provide a resilient frame or piston elements with desired shapes. For example, sections may be removed from a block of material to provide the desired shapes. Another option for preparing the piston element is 3D printing.

In a preferred embodiment the actuating piston element, the passive piston element and the resilient frame are provided as a single piece of material in its final shape. Such a material may be provided by injection moulding, e.g. of a TPE. It is especially preferred that the piston assembly is injection moulded as a single piece in its final shape from a TPE having a Shore A hardness in the range of 50 to 90, e.g. 70 to 90. This piston assembly is appropriate for an injector not containing a lubricant, e.g. a silicone lubricant.

The compressible section has a volume calculated from the inner surface of the actuating piston element facing the compressible section to the inner surface of the passive piston element facing the compressible section. The compressible section will have a volume and the part of the volume occupied by the resilient frame is, in the context of the invention, referred to as the "volumetric fraction". The resilient frame has a volumetric fraction in the range of 15% to 50% of the volume of the compressible section. For example, the resilient frame may have a generally cylindrical shape with one or more holes or indentations, which together with elastic properties of the material of the frame provide the resilience. In certain embodiments a cylindrically shaped frame has a single through-going hole. The through-going hole may have any cross-sectional shape, e.g. round, oval, rectangular, square, etc. A through-going hole will provide the frame with walls, or "weakened sections" providing resilience to the frame. When the frame comprises a through-going hole the volumetric fraction of the resilient frame will typically be in the range of 30% to 70%. In a specific embodiment the resilient frame has an axial length in the range of 30% to 70% of the total length of the piston assembly.

The resilient frame may also take the form of one or more pillars or stems linking the actuating piston element to the passive piston element. In the context of the invention the terms "stem" and "pillar" may be used interchangeably. The pillars may be designed freely, but the width, thickness and length of the pillars may vary in order to achieve the necessary contraction, which may depend on the dimension and inner diameter of the cylinder affecting the force and friction requirements for a given piston assembly. By pushing the actuating piston element the pillar(s) will deform to provide resilience to the frame. In an embodiment the pillar is or the pillars are of a linear shape. The pillar or pillars may extend at a right angle between the actuating piston element and the passive piston element, or the pillar or pillars may extend at an angle deviating from a right angle. When the angle of the pillar(s) deviate(s) from a right angle deformation of the pillar(s) is generally smoother than when the pillar(s) are at a right angle thereby providing a more user friendly operation of the injector. In a specific embodiment the pillar is curved or contains an angle. A curved or angled pillar will also deform more smoothly to provide a more user friendly operation of the injector. In another embodiment the resilient frame has a helical shape. A helically shaped resilient frame will also provide a more user friendly operation of the injector.

In a further embodiment the compressible section comprises frame in the form of a sponge or the like. For example, the material, e.g. an elastic material, of the resilient frame may have a network of a regular or irregular structure with the material making up from 20% to 40% of the total volume of the structure. A piston assembly having a frame with such a structure may be prepared by two-component moulding.

In an embodiment of the invention the actuating surface of the actuating piston element may comprise a cavity for interaction with the tip of a plunger or rod for the traditional prefilled syringes with a permanent plunger attached to the piston. To accommodate easy and simple mounting of a plunger or rod a further embodiment comprises cavities, e.g. identical cavities, at the actuating surface of the actuating piston element and the outlet surface of the passive piston element. This eliminates the need for orientation of the piston assembly upon insertion into the cylinder due to the symmetric design.

In a further embodiment the actuating piston element comprises an engagement device for engaging a complementary engagement device of a piston rod. The piston assembly preferably has a resilient frame linking the actuating piston element to the passive piston element. Thereby the injector may be used as a conventional injector allowing the user to fill the injector with a pharmaceutical composition for injection into a patient. In a specific embodiment the injector also comprises a piston rod having a complementary engagement device, i.e. complementary to the engagement device of the actuating piston element. An exemplary set of an engagement device and a complementary engagement device comprise an internal and an external helical thread. When the injector has a piston assembly with an actuating piston element having an engagement device for engaging a complementary engagement device of a piston rod, the effect of the piston assembly on the BLF is obtained both when pushing and pulling the piston assembly using the piston rod, i.e. when emptying or filling the injector. Thereby a more accurate filling of the injector can be obtained due to the low difference between the BLF and the glide force.

In general, a compressible fluid present in the compressible section will be at a pressure corresponding to the ambient pressure of the injector. However, it is also contemplated that the injector with the two piston elements may be assembled at an increased or decreased pressure. Following assembly at a modified pressure the moveable nature of the piston elements will provide that the pressure of the compressible section adjusts to the ambient pressure.

When the piston assembly comprises the resilient frame it is preferred that the piston assembly is symmetrical with respect to the actuating surface of the actuating piston element compared to the outlet surface of the passive piston element. Thereby insertion of the piston assembly in the cylinder is not dependent on its orientation. This simplifies the manufacturing of the injector compared to injectors having a piston rod engaging a piston with means for receiving the piston rod.

The injector may be any kind of injector employed to deliver a pharmaceutical composition to a subject through the skin of the subject. For example, the injector may be a syringe, which is fitted with a hypodermic needle to inject a pharmaceutical composition, e.g. via subcutaneous (SC), intramuscular (IM), intra-dermal (ID), or intravenous (IV) delivery or another type of delivery.

The injector may comprise a needle cap having a tubular section for actuating the actuating piston element, which needle cap has a needle insertion end comprising an engagement device for engaging a complementary engagement device of the outlet of the cylinder when the needle cap is mounted on the cylinder, which needle cap has a length, which is equal to or larger than an operating length of the cylinder defined by the distance from the actuating end of the cylinder to the outlet end of the cylinder minus the dimension of the piston assembly parallel with the longitudinal axis, e.g. in a relaxed state. In general, the distance from the actuating end of the cylinder to the outlet end of the cylinder minus the dimension of the piston assembly parallel with the longitudinal axis of the cylinder defines an "operating length" of the cylinder. The piston assembly may be moved towards the outlet end using any means from the actuating end, for example the piston assembly may be moved towards the outlet end using a piston rod or a plunger. It is preferred that the piston assembly cannot be moved towards the actuating end, e.g. from the outlet end, with engagement of the piston assembly, e.g. with a piston rod or the like, from the actuating end. The needle cap may consist of the elastomeric material or it may consist of a rigid material, e.g. a polymeric rigid or elastomeric material. Further details on this embodiment are found in US 2016/0129197 and DK 178284 B1, in particular, the respective claims.

The cylinder at the actuating end may be open across the whole cross-section of the cylinder, which allows for removal and insertion of the piston assembly and thereby also filling of the injector via the actuating end. The cylinder may also have at the actuating end a ridge or protrusion(s) or the like preventing removal of the piston assembly once inserted in the cylinder. In particular, the ridge or protrusion(s) may provide a "lock device" of a "spring-lock device" where the complementary "spring device" is contained on a piston rod. A spring-lock device or the like can lock the piston rod after moving the piston assembly to the outlet end of the cylinder thereby preventing refilling of the cylinder.

The injector may comprise, e.g. at the outlet end, a fitting for attaching or mounting a hypodermic needle. The cylinder may thus have a tapered outlet, e.g. a tubular outlet, from the cylinder providing an engagement device for engaging a complementary engagement device of a hypodermic needle, e.g. the engagement device and the complementary engagement device may comprise a male-female interaction, with the tubular outlet optionally comprising an external thread, e.g. a helical external thread, and the hypodermic needle optionally comprising a complementary internal thread, e.g. a helical internal thread. A hypodermic needle may be fitted to allow simple removal, and replacement, of the hypodermic needle, or the hypodermic needle may be mounted permanently on the injector. In particular, the hypodermic needle may be mounted on the injector so that its removal requires the destruction of the injector thereby preventing reuse, which in the context of the invention is considered "permanent". It is preferred that the injector comprises a hypodermic needle attached, e.g. permanently attached, to the outlet of the cylinder.

In an embodiment of the invention the injector, preferably prefilled, is a syringe with a hypodermic needle. The syringe may have a hypodermic needle mounted, e.g. permanently mounted, on a tubular outlet or an outlet of another shape. When the injector is prefilled, in particular when it also comprises a needle cap for use as a piston rod, there may be a clearance between the actuating end of the cylinder and the actuating surface of the piston assembly. The clearance ensures stability of a piston rod when this is inserted in the cylinder, which results in a safer and easier operation of the injector. The clearance, e.g. measured in units of length, may be any value relevant for the size, e.g. volume, of injector and the dose of pharmaceutical composition in the injector. Typical values for the clearance are between about 2 mm to about 20 mm. However, the clearance may be in excess of 20 mm in cases where the actual volume of the injectable volume is considerably less than the cylinder usable volume, e.g. for ophthalmic injections where the injectable volume is only 0.05 ml, although the injector body and hence the cylinder is considerably larger and especially longer in order for the user to be able to handle and control the injector

In an embodiment of the invention the actuating piston element, the passive piston element and/or the resilient frame comprise one or more stabilising fins. A stabilising fin may take any form or shape as desired and will typically provide a non-sealing contact between the respective piston element or the resilient frame and the inner wall of the cylinder. A stabilising fin will generally be present on the respective piston element at a location different from the abutting surface between the piston element and the inner wall of the cylinder, e.g. between the actuating surface and the deformable sealing element of the actuating piston element or between the outlet surface and the deformable sealing element of the passive piston element, or as part of the resilient frame when present. In an embodiment the stabilising fin is a circular outward protrusion surrounding a body of the piston element, which body does not contact the inner wall of the cylinder, and the stabilising fin is positioned between the inner surface of the respective piston element and the section of the respective piston element abutting the inner wall of the cylinder, e.g. a convex deformable sealing element, or between the section of the respective piston element abutting the inner wall of the cylinder and the resilient frame, or on the resilient frame. When a stabilising fin, e.g. in the shape of a circular outward protrusion, is positioned between the inner surface of the respective piston element and the section of the passive piston element abutting the inner wall of the cylinder it is ensured that there is a lower risk of residual injectable substance left after completed injection compared to the embodiment having a stabilising fin between the section of the passive piston element abutting the inner wall of the cylinder and the outlet surface. The stabilising fins may also be present at a location between the deformable sealing element and the compressible section of either piston element. In an embodiment, the stabilising fins have a longitudinal direction relative to the cylinder into which the piston elements are inserted. The stabilising fins therefore contribute only marginally to the BLF of piston assembly. Regardless of its position on a piston element the stabilising fin prevents tilting of the respective piston element. Prevention of tilting provides a more robust injector. When a piston in an injector tilts the operation of the injector becomes less predictable, and in particular, in embodiments of the injector of the present invention, in which a compressible fluid is present in the compressible section tilting can cause a malfunction of the injector with respect to lowering the BLF. Therefore, stabilising fins are especially advantageous to the injector of the invention.

In a specific embodiment the stabilising fin is flexible, in particular in an axial direction of the cylinder of the injector. A flexible stabilising fin will typically comprise one or more sections surrounding the piston body and have a radial extension, which is larger than the inner diameter or the distance, e.g. the radial distance, between the inner walls of the cylinder. For example, the flexible stabilising element can have a diameter, or radial dimension, in the range of 120% to 200% of the inner diameter, or distance between the inner walls, of the cylinder. Upon insertion into the cylinder the flexible stabilising fin will bend and slide along the inner wall of the cylinder without significantly contributing to the glide force of the piston assembly. Once inserted into the cylinder the flexible stabilising fin of a piston assembly having a flexible stabilising fin will only provide an insignificant contribution to the BLF of the piston assembly. The flexible stabilising fin, e.g. a flexible stabilising fin having a radial extension, which is larger than the inner diameter of the cylinder of the injector, will improve the stability against tilting of the piston assembly. A stabilising fin may for example take the form of a collar surrounding the piston body, or the stabilising fin may comprise two or more, e.g. three or four, flaps, radially extending from a centre axis of the piston body. The thickness of the flexible stabilising fin, e.g. in an axial direction of the piston body, will typically be up to 1 mm, e.g. in the range of 0.1 mm to 1.0 mm. The flexible stabilising fin preferably has a Shore A hardness in the range of 50 to 90, and it is more preferably made from the same material as the piston body and/or the piston elements. In an embodiment the piston assembly including a flexible stabilising fin is injection moulded as a single piece, e.g. from a TPE. An axially flexible stabilising fin is especially appropriate when the compressible section consists of a compressible fluid since tilting of either the actuating piston element or the passive piston element will prevent sealing the compressible section.

The features of the injectors of any aspects of the invention may be combined freely and any advantage obtained for a specific feature is available to either aspect by incorporated the respective feature in the injector.

### Brief description of the figures

In the following the invention will be explained in greater detail with the aid of examples and with reference to the schematic drawings, in which
Figure 1 shows a perspective view of a piston of the prior art;
Figure 2 shows a perspective view of a piston assembly of an injector of the invention;
Figure 3 shows a side view of a piston assembly of an injector of the invention;
Figure 4 shows side views of an injector of the invention;
Figure 5 shows side views of an injector of the invention;
Figure 6 shows a side view of a piston assembly of an injector of the invention;
Figure 7 shows a side view of a piston assembly of an injector of the invention;
Figure 8 shows a top view of the piston assembly of an injector shown in Figure 7;
Figure 9 shows a perspective view of a piston assembly of an injector of the invention;
Figure 10 shows a side view of the piston assembly shown in Figure 9;
Figure 11 shows a side view of an injector with the piston assembly of Figure 9 and Figure 10;
Figure 12 shows side views of a piston assembly and an injector of the invention;
Figure 13 shows side views of a piston assembly and an injector of the invention;
Figure 14 shows side views of a piston assembly and an injector of the invention;
Figure 15 shows side views of an injector of the invention;
Figure 16 shows a perspective view of a piston assembly of an injector of the invention;
Figure 17 shows a perspective view of a piston assembly of an injector of the invention;
Figure 18 shows a perspective view and side views of a piston assembly of an injector of the invention;
Figure 19 shows a side view of a piston assembly of an injector of the invention;
Figure 20 shows a perspective view of a piston assembly of an injector of the invention with a piston rod;
Figure 21 shows a perspective view of a piston assembly of an injector of the invention;
Figure 22 shows different views of a piston assembly of an injector of the invention;
Figure 23 shows different views of a piston assembly of an injector of the invention;
Figure 24 shows a plot of load vs. displacement for an injector of the prior art.
Figure 25 shows a plot of load vs. displacement for an injector of the invention.

It should be understood that combinations of the features in the various embodiments are also contemplated, and that the various features, details and embodiments may be combined into other embodiments.

Reference to the figures serves to explain the invention and should not be construed as limiting the features to the specific embodiments as depicted.

### Detailed description of the invention

The present invention relates to injectors for delivery of a pharmaceutical composition. The present invention will now be described in greater detail with reference to the appended drawings. Certain figures are depicted as "cross-sectional views" of the injectors of the invention, where the injector in the "cross-sectional view" is depicted at an angle of 90° compared to the injector otherwise depicted. Certain figures depict side views of injectors of the invention. These side views do not depict the outlet of the injectors but it is to be understood that the injector of the invention will have an outlet, e.g. fitted with a hypodermic needle.

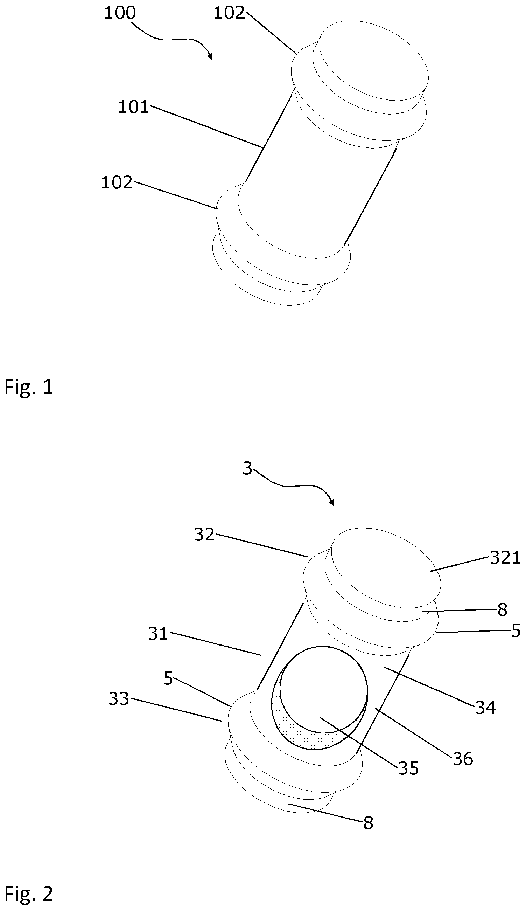

Figure 1 shows a conventional piston 100 of a syringe (not shown) for injection of a pharmaceutical compound. The conventional piston comprises a piston body 101 and, in the version shown, two sealing elements 102, which, when the conventional piston 100 is inserted into a syringe, seal an annular gap between the piston body 101 and the inner wall of the syringe. The piston body 101 is typically made of rubber or a similar material with low compressibility. In order to move the conventional piston 100 in the syringe the break loose force (BLF) will include the combined static friction of the two sealing elements 102.

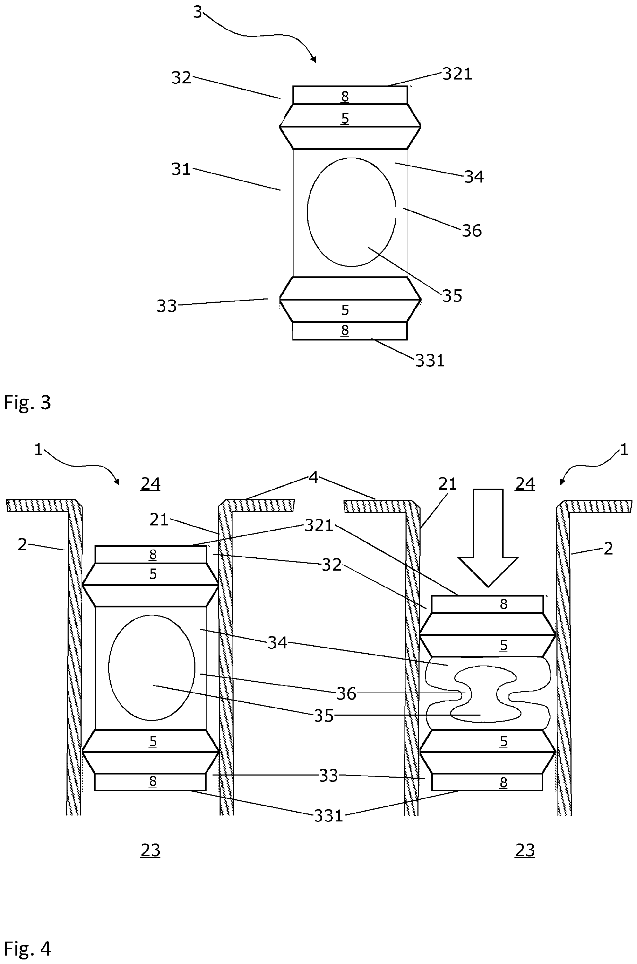

Figure 2 shows a perspective view of a piston assembly 3 having a compressible section 31 between an actuating piston element 32 and a passive piston element 33. The actuating piston element 32 comprises a resilient frame 34, which in the embodiment shown, has a single through-going hole 35 and corresponding walls 36. The actuating piston element 32 has an actuating surface 321, and the passive piston element 33 has an outlet surface 331; in the embodiment shown the piston assembly 3 is symmetrical relative to the actuating surface 321 and the outlet surface 331 so that orientation of the piston assembly 3 in the injector is irrelevant. The piston elements 32,33 of the piston assembly 3 of Figure 2 have convex deformable sealing elements 5, and the piston assembly 3 has been prepared as a single piece by injection moulding of a thermoplastic elastomer (TPE), e.g. having a Shore A hardness in the range of 50 to 90. The piston assembly 3 of Figure 2 is depicted as a side view in Figure 3, and in Figure 4 the piston assembly 3 is shown inserted into an injector 1. Figure 4 shows the actuating end 24 of the cylinder 2 where finger grips 4 are located. The convex deformable sealing elements 5 of the piston elements 32,33 abut the inner wall 21 of the cylinder 2 and provide seals between the piston assembly 3 and the inner wall 21. In Figure 4 (left panel) the piston assembly 3 is shown in a relaxed state and in Figure 4 (right panel) a force (indicated by the arrow) is applied to the actuating surface 321 so that the compressible section 31 is compressed; the walls of the resilient frame 36 are deformed by the compression. The axial length of the compressible section 31 relative to the total length of the piston assembly 3 (in a relaxed state) is about 50%, and the volumetric fraction of the resilient frame 34 is about 30% in Figure 2 to Figure 4.

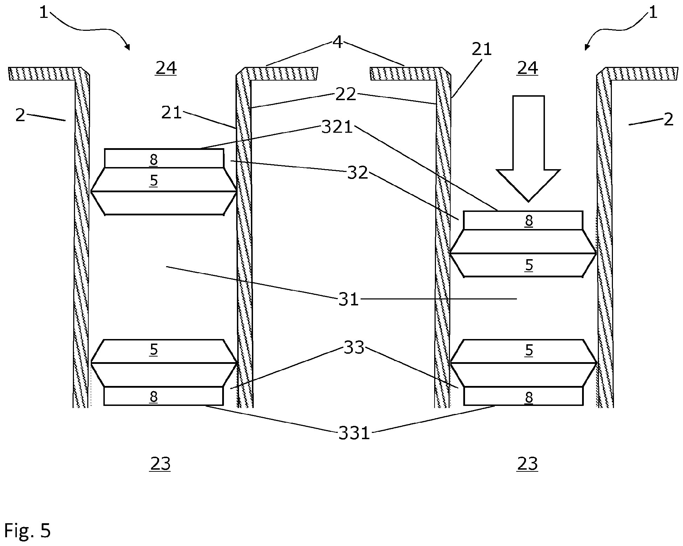

Figure 5 shows an embodiment where the compressible section 31 consists of a compressible fluid, e.g. air. Thus, the piston assembly 3 comprises an actuating piston element 32 and a passive piston element 33, which are both made from an Evoprene having a Shore A hardness of 71, and it does not comprise the resilient frame. The actuating piston element 32 and a passive piston element 33 may be made from any TPE having a Shore A hardness in the range of 50 to 90, e.g. any TPE listed in Table 1. In Figure 5 (left frame) the piston assembly 3 is shown in a relaxed state and in Figure 5 (right frame) a force (indicated by the arrow) is applied to the actuating surface 321 so that the compressible section 31 of the compressible fluid is compressed.

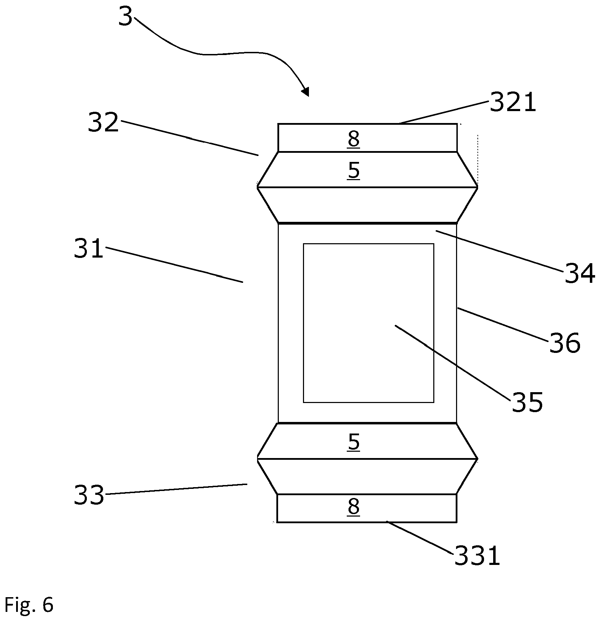

The through-going hole 35 is depicted as having a round cross-section in Figure 2 and Figure 3, but the through-going hole is not limited with respect to the cross-sectional shape. Thus, Figure 6 shows an embodiment where the through-going hole 35 has a rectangular cross-section. In other embodiments the resilient frame 34 can have any number of through-going holes of any cross-sectional shape. It is moreover possible for the resilient frame 34 to have one or more indentations, e.g. holes that are not through-going.

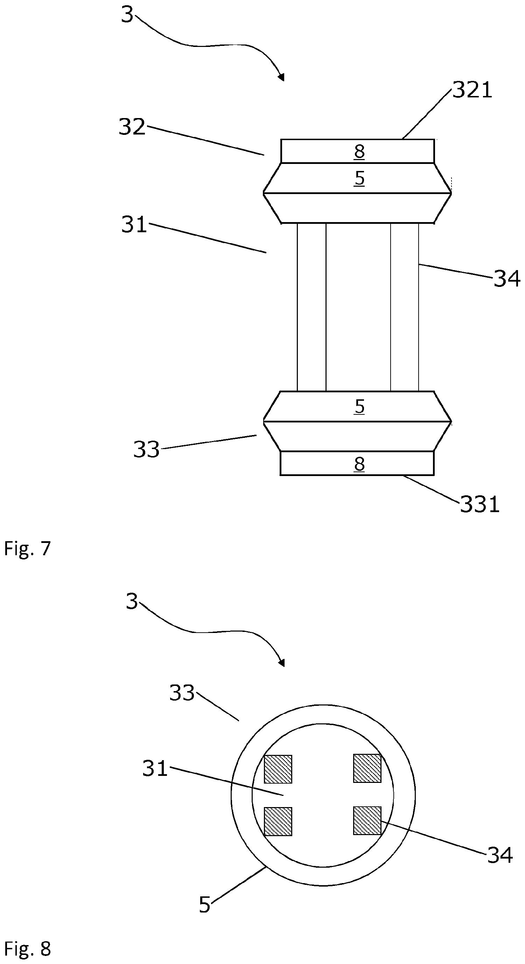

Figure 7 to Figure 14 show embodiments where the resilient frame 34 has the form of one or more pillars 37; in these embodiments the resilient frame 34, the actuating piston element 32 and the passive piston element 33 are typically prepared from the same material, e.g. by injection moulding of a TPE.

Figure 7 shows an embodiment where the resilient frame 34 consists of four pillars 37; in Figure 8 the four pillars 37 are shown in a (cross-sectional) topview. The pillars 37 have a square cross-section and extend at right angles between the inner surfaces 311 of the piston elements 32,33. The volumetric fraction of the resilient frame 34 is in the range of 15% to 25% in the embodiment of Figure 7 and Figure 8.

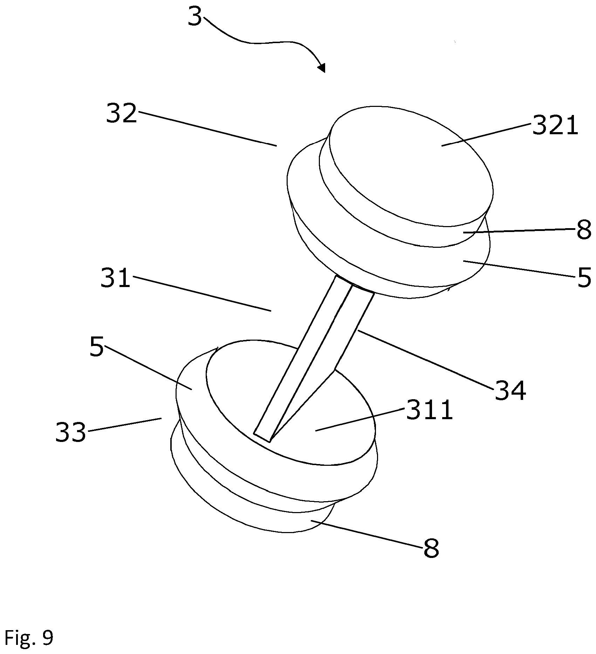

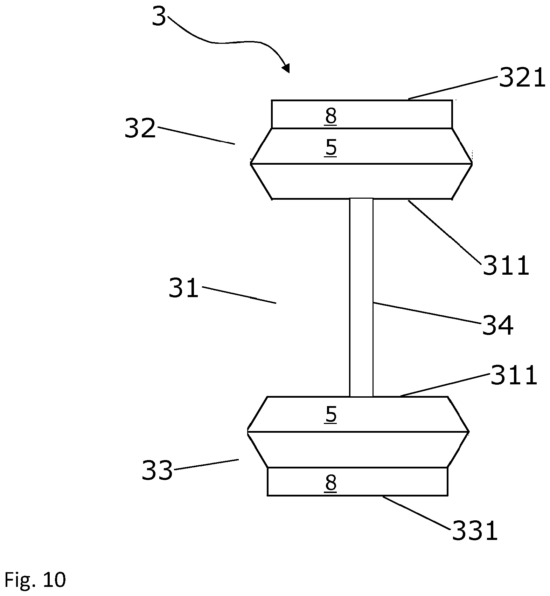

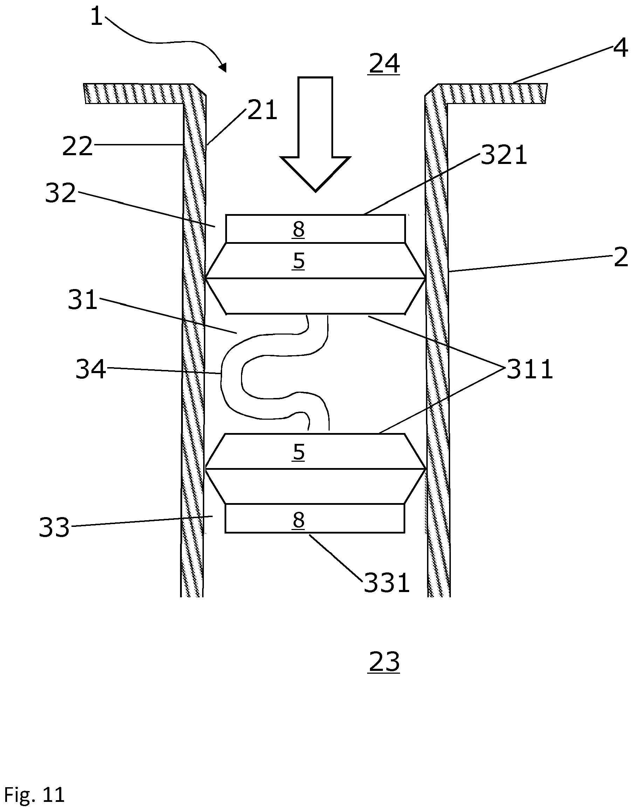

Figure 9 shows a perspective view of a piston assembly 3 having a resilient frame 34 consisting of a single pillar, which is shown as a side view in Figure 10. The pillar extends at right angles between the inner surfaces 311 of the piston elements 32,33, and Figure 11 shows the piston assembly 3 inserted into a cylinder 2 and deformed by an external force (indicated by the arrow).

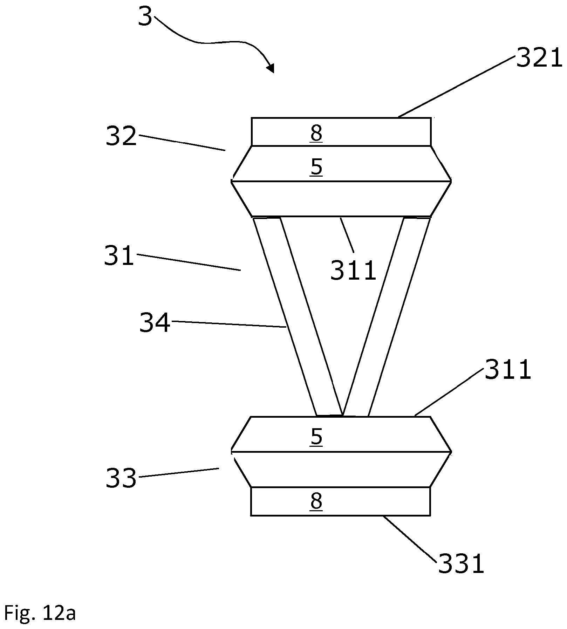

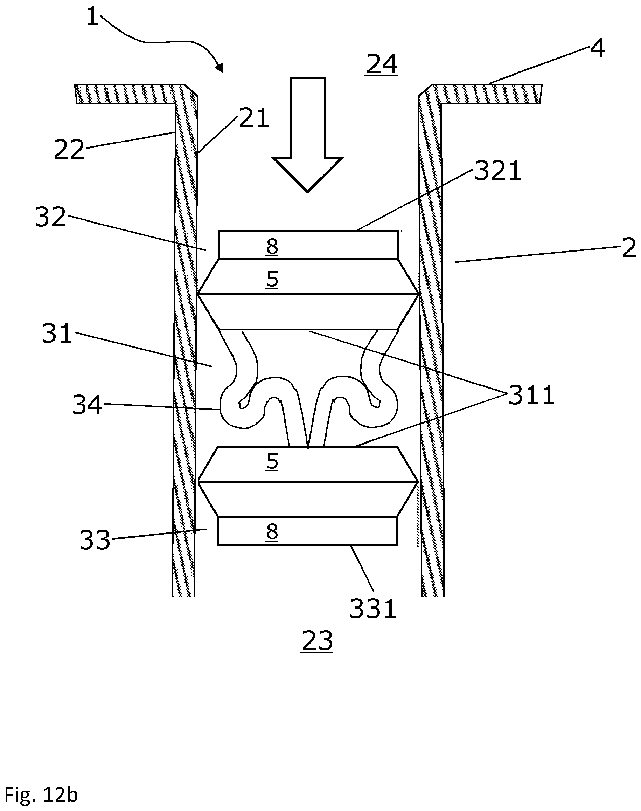

In Figure 12 the resilient frame 34 has two pillars which extend from the side of the inner surface 311 of the actuating piston element 32 to the centre of the inner surface 311 of the passive piston element 33 or vice versa. Figure 12a shows the piston assembly 3 in a relaxed state, and in Figure 12b the piston assembly 3 has been inserted into the cylinder 2 and is shown in a compressed state where the pillars are deformed by application of an external force as indicated by the arrow. It is noted that Figure 12b shows the piston assembly 3 in one orientation with respect to the cylinder 2 but that this embodiment is considered symmetrical with respect to the actuating surface 321 and the outlet surface 331 of the respective piston elements 32,33 and that the orientation in the cylinder 2 is not relevant for correct functioning of the piston assembly 3.

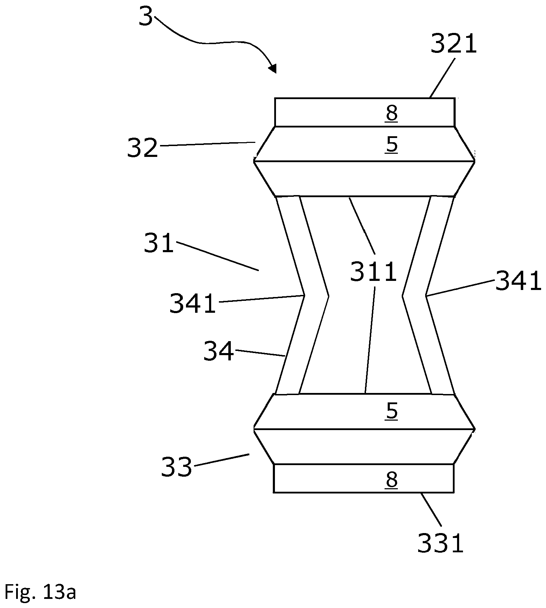

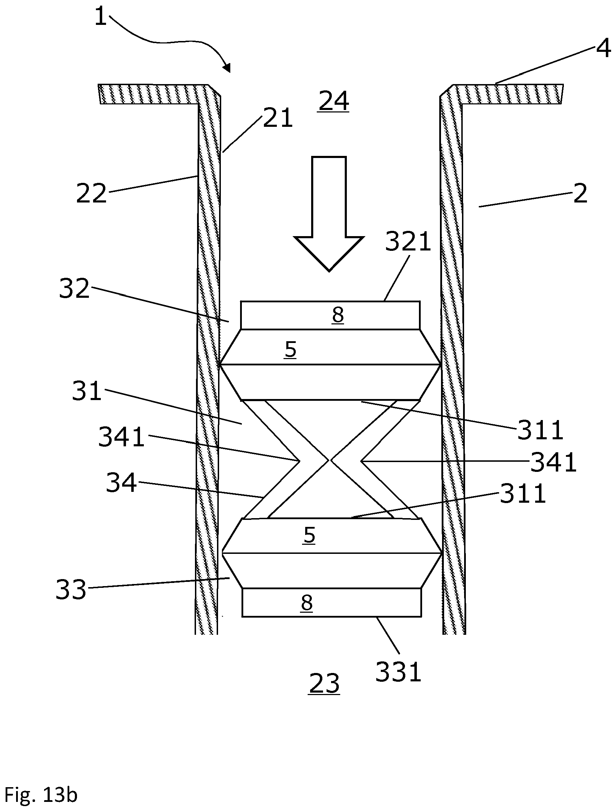

Figure 13 shows side views of a piston assembly 3 (Figure 13a) and a cylinder 2 containing the piston assembly 3 (Figure 13b), which piston assembly 3 has a resilient frame 34 in the form of two pillars each containing an angle 341. The pillars are made from a TPE but could, due to the angle 341, also be prepared from a more rigid material, e.g. a polymer. The angle 341 provides resilience to the resilient frame 34, and upon exposure to an external force as depicted in Figure 13b the angle 341 will bend allowing the actuating piston element 32 to compress the compressible section 31 and then eventually push the passive piston element 33 at a reduced BLF compared to the added BLF of each of the piston elements 32,33.

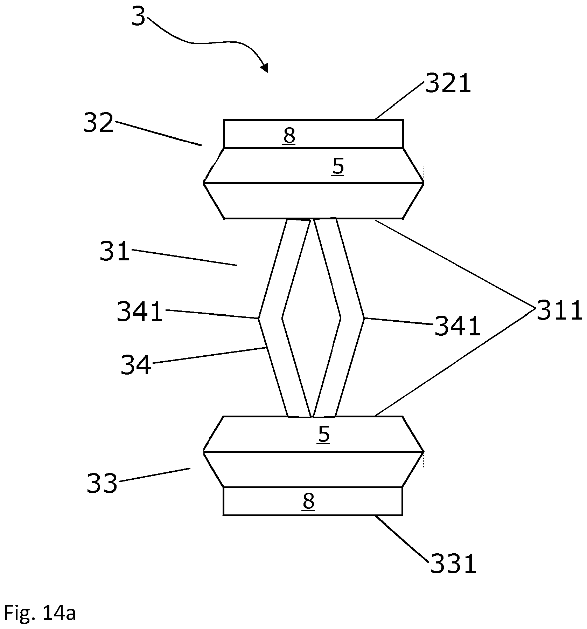

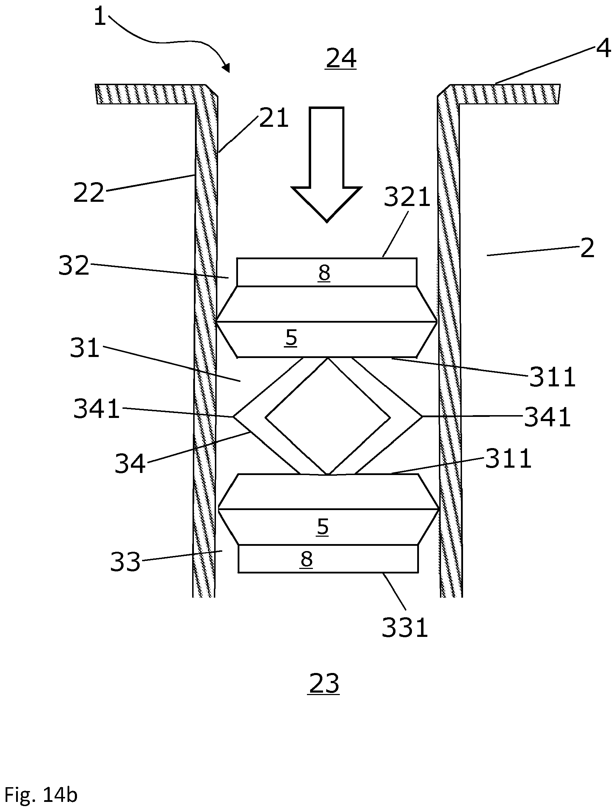

Figure 14 shows a variation of the embodiment depicted in Figure 13 with Figure 14a showing the piston assembly 3 in a relaxed state and Figure 14b showing the piston assembly 3 in a cylinder 2 compressed by an external force (indicated by an arrow) where the angles 341 of the pillars making up the resilient frame 34 are mirrored compared to the angles 341 of the embodiment in Figure 13. All observations made for the embodiment in Figure 13 are relevant also for the embodiment of Figure 14.

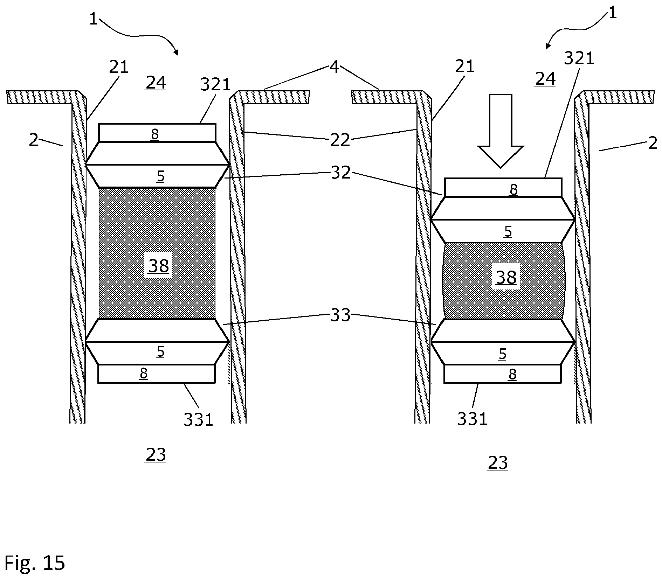

Figure 15 shows side views of an injector 1 having piston element 3 with a compressible element 31 made from a spongy material 38. Figure 15 (left panel) shows the piston element 3 in a relaxed stated and in Figure 15 (right panel) the spongy material 38 is compressed by an external force (indicated by an arrow) so that the compressible section 31 is also compressed.

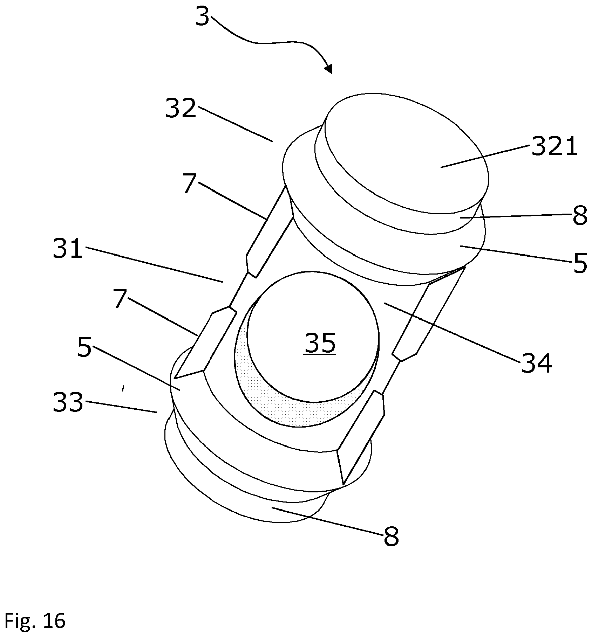

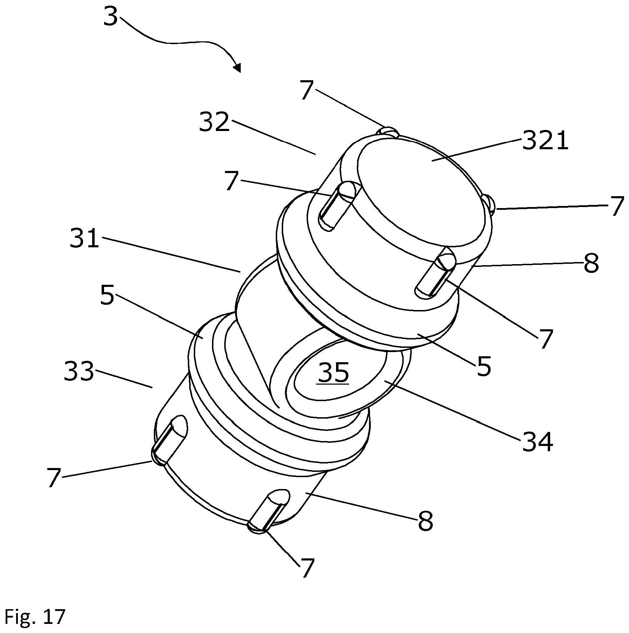

Figure 16 and Figure 17 show embodiments where the resilient frame 34 and the piston elements 32,33, respectively, are provided with stabilising fins 7. The piston elements 32,33 each have a piston body 8, which will not contact the inner wall 21 of the cylinder 2 once the piston assembly is inserted in the cylinder 2. The stabilising fins 7 ensure that the piston assembly 3 can be inserted into the cylinder 2 and that the piston assembly 3 can be deformed without risk of tilting of a piston element 32,33. The stabilising fins 7 are present as longitudinally extended protrusions on the resilient frame 34 and/or the piston elements 32,33 where they provide a non-sealing contact with the inner wall of the cylinder.

The embodiment depicted in Figure 17 has a cylindrically shaped resilient frame 34 transverse to the cylinder. This embodiment of the piston assembly 3 may be prepared by gluing or welding appropriately shaped piston elements 32,33 with the resilient frame 34, or the piston assembly 3 including the piston elements 32,33 and the resilient frame 34 may be injection moulded as a single piece.

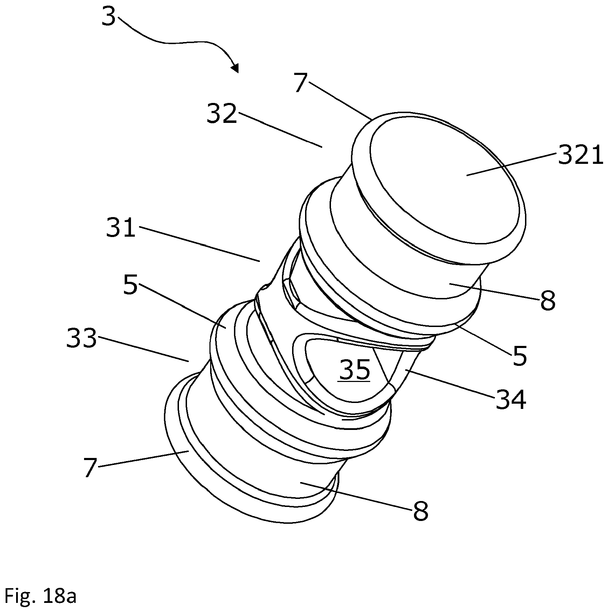

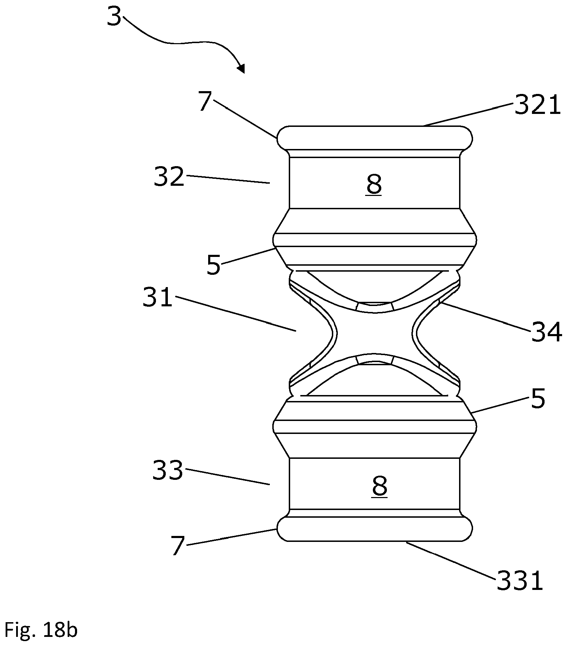

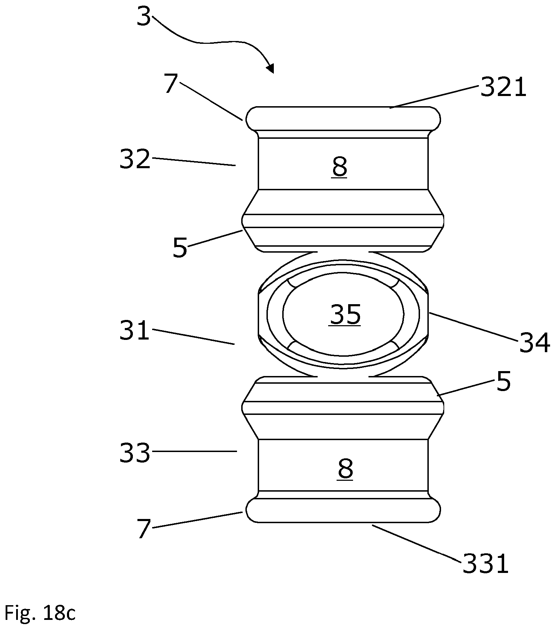

Figure 18 shows an embodiment of the piston assembly 3, where the actuating piston element 32 and the passive piston element 33 each have stabilising fins 7 in the form of circumferential structures but otherwise providing the function of the stabilising fins 7 described above. The piston assembly 3 is depicted in a perspective view in Figure 18a and in side views at different right angles in Figure 18b and Figure 18c.

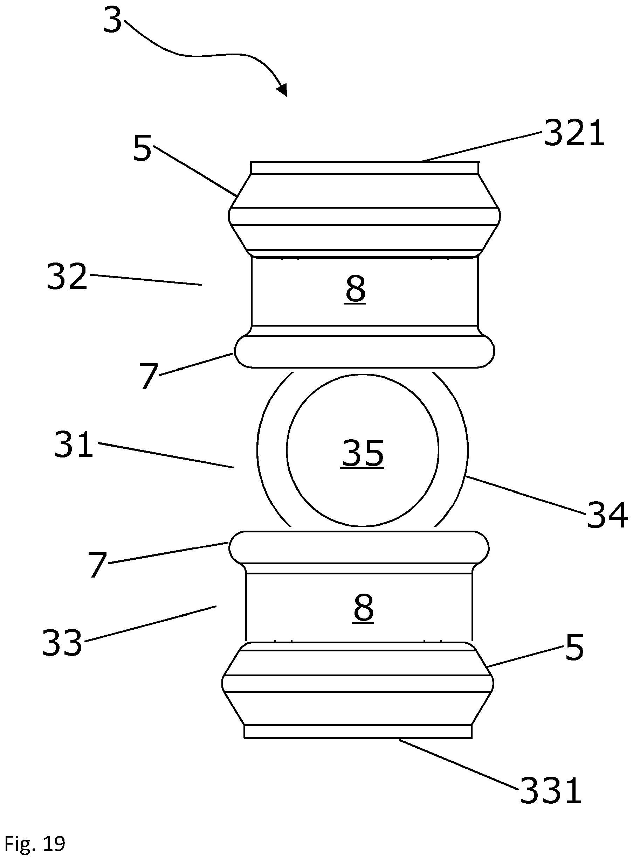

Figure 19 shows a side view of an embodiment of the piston assembly 3 where a circular stabilising fin 7 surrounds a piston body 8, which will not contact the inner wall 21 of the cylinder 2 once the piston assembly is inserted in the cylinder 2. The stabilising fin 7 is located between the section of the respective piston elements 32,33 abutting the inner wall 21 of the cylinder 2, once the piston assembly is inserted in the cylinder 2, and the resilient frame 34.

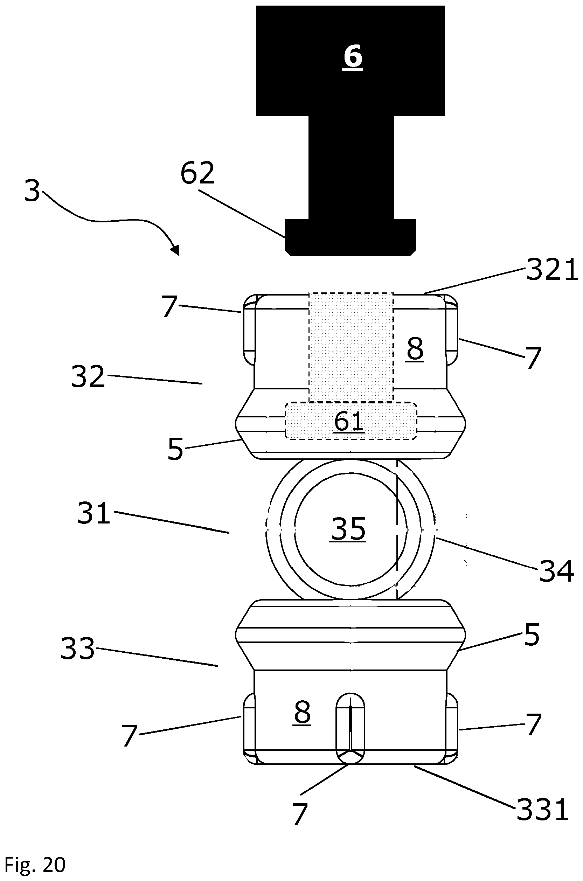

Figure 20 shows a side view of an embodiment of the piston assembly 3 where the actuating piston element 32 has an engagement device 61 for engaging a complementary engagement device 62 of a piston rod 6. When the piston rod 6 engages the actuating piston element 32 via the engagement device 61 and the complementary engagement device 62 it is possible to fill the injector with a pharmaceutical composition for injection into a patient. For ease of automatic assembly of the injector 1, e.g. with regard to the piston assembly 3 shown in Figure 20, the passive piston element 33 may have a similar engagement device (not shown) enabling the omitting of piston orientation during production.

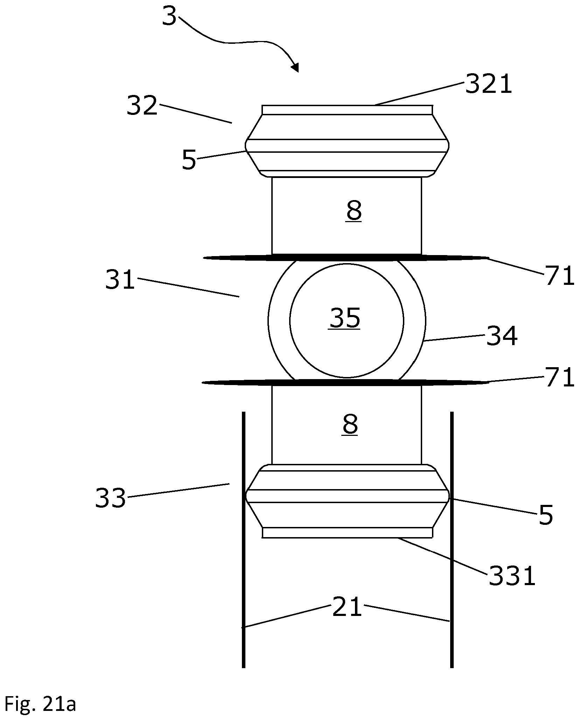

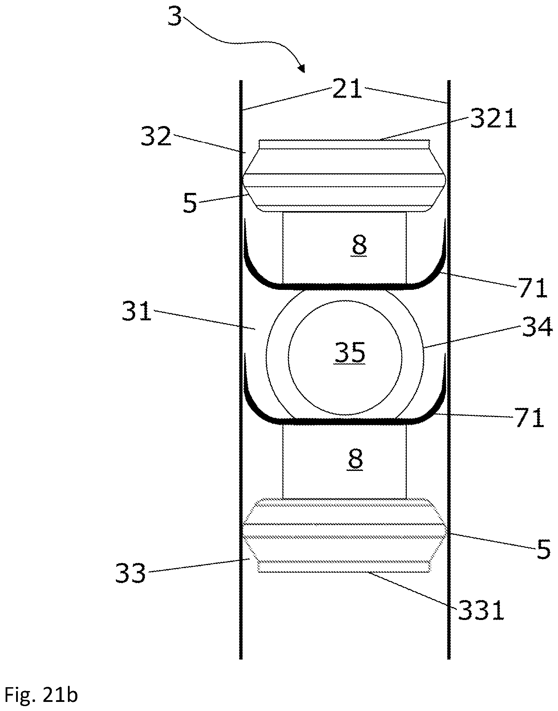

Figure 21 shows a side view of an embodiment of the piston assembly 3 where the piston body 8 has a flexible stabilising fin 71. The flexible stabilising fins 71 of the embodiment shown are a made from single pieces of TPE fully surrounding the piston body 8. However, a flexible stabilising fin 71 may also comprise more than one, e.g. three, four or more, sections, for example in the form of flaps. In Figure 21a the piston assembly 3 is shown partly inserted into a cylinder illustrated with the inner walls 21, and in Figure 21b the piston assembly 3 is shown fully inserted between the inner walls 21. The radial extension, e.g. the diameter, of the flexible stabilising fin 71 is larger than the inner diameter of the cylinder, so that when the flexible stabilising fin 71 is inserted into the cylinder the flexible stabilising fin 71 will bend along the inner walls 21 of the cylinder in a direction opposite the direction of the movement of the piston assembly 3 relative to the cylinder.

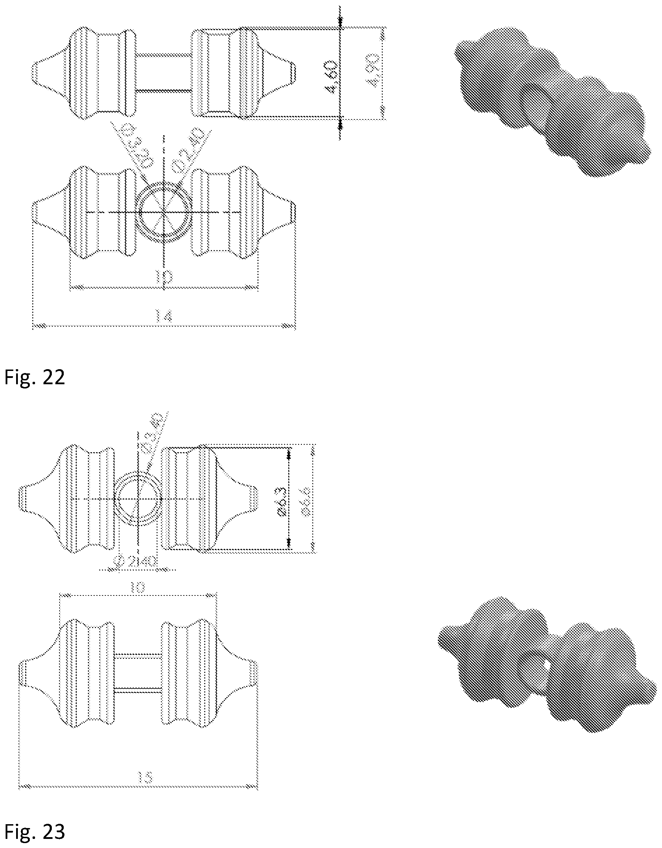

Figure 22 and Figure 23 show embodiments of piston assemblies made for glass cylinders of 0.5 mL and 1.0 mL volume, respectively. The piston assemblies are injection moulded as single pieces from Evoprene and the piston assemblies are symmetrical with respect to the actuating surface and the outlet surface of the respective piston elements so that no orientation of the piston assemblies is required when inserting into the cylinder. The actuating surface and the outlet surface have a protrusion each providing a specific function. The protrusion of the outlet surface is shaped to ensure complete emptying of the cylinder upon injection, and the identical protrusion of the actuating surface has a shape complementary to the shape of a needle cap functioning as a piston rod, e.g. a tubular section for housing the hypodermic needle as described in US 2016/0129197 and DK 178284 B1. Figure 22 and Figure 23 show side views of the piston assemblies where one view illustrates the cylindrical resilient frame, which is depicted at a 90° rotation in the other view; the piston assemblies are also shown in perspective views also depicting the cylindrical resilient frames. Measures, in mm, are indicated in Figure 22 and Figure 23.

### Examples

### Example 1

The container closure integrity of an injector of the invention was tested. Glass syringes with an integrated hypodermic needle were filled with a blue dye solution prepared according to the guidelines in ASTM F 1929. Until the testing began, the injectors were stored at 23°C, 50% RH. The test components were un-siliconised or without any other lubricant. The pre-filled injectors used in this study had a piston assembly of the thermoplastic elastomer (TPE) Evoprene G970 with a diameter, in the relaxed state, of the piston elements of 4.88 mm. The nominal capacity of the injectors was 0.5 mL, and the cylinders were made from borosilicate glass.

The test was conducted by placing the injectors on absorbent paper in desiccator as follows:
- 25 mbar for 10 min, inspection, and then
- 35 mbar for 10 min, inspection, and then
- 100 mbar for 10 min, final inspection.

The results are summarised in Table 2.

**Table 2 Results of container closure integrity tests**

| No. of replicates | Inspection after -25 mbar, 10 min | Inspection after -35 mbar, 10 min | Final inspection -100 mbar, 10 min |
|---|---|---|---|
| 5 | No leaking syringes | No leaking syringes | No leaking syringes |

### Example 2

The forces required to empty an injector of the invention was tested and compared to an injector of the prior art. Pre-filled glass injectors with integrated hypodermic needles were stored at 23°C, 50% RH until testing began. The test components were un-siliconised or without any other lubricant or coating. The prefilled injectors of the invention used in this study had a piston assembly of Evoprene G970 with a diameter, in the relaxed state, of the piston elements of 4.88 mm. Specifically, the tested piston assembly of the invention is depicted in Figure 2. The prior art injector likewise had a piston of Evoprene G970. The nominal capacities of the injectors were 0.5 mL, and the cylinders were made from borosilicate glass. Testing was based on ISO 7886-3:2005 Annex B Sterile hypodermic syringes for single use - Part 3: Auto-disable syringes for fixed-dose immunisation, Test method for forces required to operate plunger. An Instron mechanical testing machine equipped with 100 N load cell was employed at 100 mm/min.

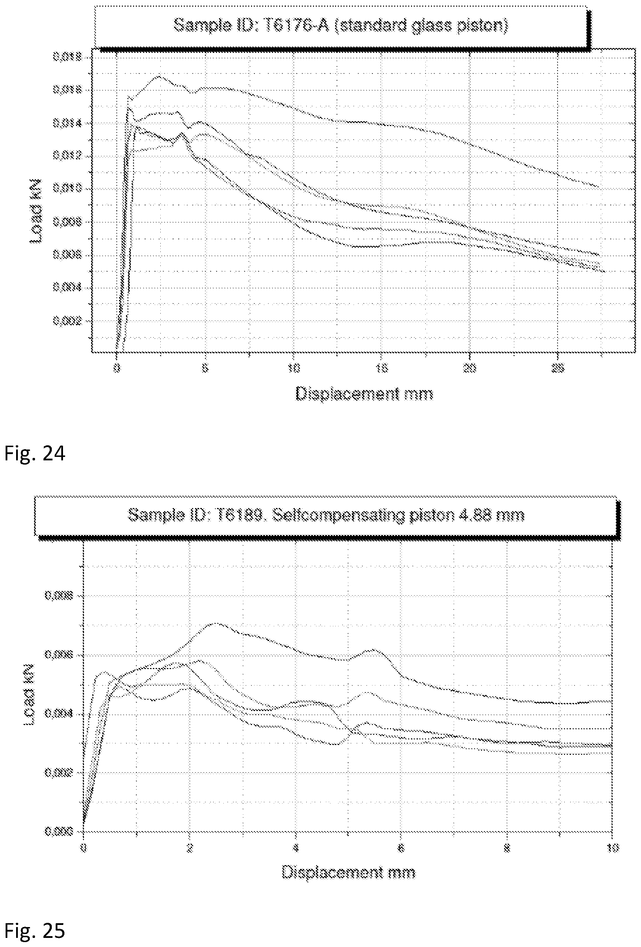

The results are summarised in Table 3 and depicted in Figure 24 (injector with a piston of the prior art) and Figure 25 (injector of the invention).

**Table 3 Results of tests of forces required to empty an injector**

| Sample | No. of replicates | Average maximum BLF (N) | Glide force (N) |
|---|---|---|---|
| Prior art | 5 | 14(1) | 5-15 |
| Injector of the invention | 5 | 6(1) | 3-6 |

It is evident from Figure 24 and Figure 25 that the injector of the invention reduced the overall BLF to a value similar to the glide force of the piston assembly. This demonstrates that the present invention provides an injector with a smooth force profile experienced by the end-user. In contrast, the injector of the prior art had a conventional force profile requiring a much higher force to initiate movement compared to retaining movement. The summarised results in Table 3 show the BLF's of the two tested injectors with the number in brackets representing the standard deviations.

## Claims

1. An injector (1) for delivery of a pharmaceutical composition, which injector (1) does not comprise a silicone lubricant, the injector (1) optionally comprising a piston rod, and which injector (1) comprises
- a cylinder (2) extending along a longitudinal axis, the cylinder (2) having an inner wall (21), an outer wall, and an outlet at an outlet end (23) opposite an actuating end (24),
- a piston assembly (3) comprising a compressible section (31) between an actuating piston element (32) having a Shore A hardness in the range of 50 to 90 and a passive piston element (33) having a Shore A hardness in the range of 50 to 90, which piston elements (32,33) abut the inner wall (21) of the cylinder (2) at abutting interfaces thereby sealing the compressible section (31) when the piston assembly (3) is inserted in the cylinder (2), **characterised in that** the compressible section (31) comprises a compressible fluid and a resilient frame (34), the resilient frame (34) physically coupling the actuating piston element (32) to the passive piston element (33), which resilient frame (34) is made from an elastic polymer, such as a thermoplastic elastomer (TPE) having a Shore A hardness in the range of 50 to 90, and has a volumetric fraction in the range of 15% to 50% of the volume of the compressible section (31) and which resilient frame (34) has an axial length in the range of 25% to 90% of the total axial length of the piston assembly (3) in a relaxed state, and **in that** the compressible fluid is entrapped between the actuating piston element (32) and the passive piston element (33).

2. The injector (1) according to claim 1, wherein the break loose force (BLF) is less than twice as high as the glide force.

3. The injector (1) according to any one of claims 1 or 2, wherein the diameter of the actuating piston element is smaller than the diameter of the passive piston element when the piston assembly is in a relaxed state.

4. The injector (1) according to any one of claims 1 to 3, wherein the actuating piston element (32), the passive piston element (33), or the resilient frame (34), or the actuating piston element (32), the passive piston element (33) and the resilient frame (34) are made from a thermoplastic elastomer (TPE).

5. The injector (1) according to claim 1, wherein the TPE has a compression set value of at least 25% at a temperature in the range of 10°C to 40°C over a period of time in the range of 18 hours to 96 hours according to the ISO 815 standard.

6. The injector (1) according to any one of claims 1 to 5, wherein the cylinder (2) has an inner diameter, and the actuating piston element (32) and the passive piston element (33) each comprise a convex deformable sealing element having a diameter 3% to 20% larger than the inner diameter of the cylinder (2).

7. The injector (1) according to any one of claims 1 to 6, wherein the actuating piston element (32), the passive piston element (33) and the resilient frame (34) are injection moulded as a single piece.

8. The injector (1) according to any one of claims 1 to 7, wherein the piston assembly (3) is symmetrical with respect to an actuating surface (321) of the actuating piston element (32) compared to an outlet surface (331) of the passive piston element (33).

9. The injector (1) according to any one of claims 1 to 8, wherein the injector (1) comprises a piston rod (6), and the actuating piston element (32) comprises an engagement device (61) for engaging a complementary engagement device (62) of the piston rod (6).

10. The injector (1) according to claim 8, wherein the actuating piston element (32) and the passive piston element (33) do not comprise an engagement device (61) for engaging a complementary engagement device (62) of a piston rod (6).

11. The injector (1) according to any one of claims 1 to 10, wherein the cylinder (2) is made from glass, a polymeric material, a metal, or any combination of polymeric materials, glasses or metals.

12. The injector (1) according to any one of claims 1 to 11, wherein the actuating piston element (32), the passive piston element (33) and/or the resilient frame (34) comprise one or more stabilising fins (71).

13. The injector (1) according to any one of claims 1 to 12, wherein the injector (1) comprises a needle cap having a tubular section for actuating the actuating piston element (32), which needle cap has a needle insertion end comprising an engagement device for engaging a complementary engagement device of the outlet of the cylinder (2) when the needle cap is mounted on the cylinder (2), which needle cap has a length, which is equal to or larger than an operating length of the cylinder (2) defined by the distance from the actuating end (24) of the cylinder (2) to the outlet end (23) of the cylinder (2) minus the dimension of the piston assembly (3) parallel with the longitudinal axis.

14. The injector (1) according to any one of claims 1 to 13, wherein the cylinder (2) has an inner diameter in the range of 2 mm to 10 mm.

15. The injector (1) according to any one of claims 1 to 14, wherein the cylinder (2) is prefilled with a pharmaceutical composition.

## Patentansprüche

1. Injektor (1) zur Abgabe einer pharmazeutischen Zusammensetzung, wobei der Injektor (1) kein Silikonschmiermittel umfasst, wobei der Injektor (1) wahlweise eine Kolbenstange umfasst und wobei der Injektor (1) Folgendes umfasst:
- einen Zylinder (2), der sich entlang einer Längsachse erstreckt, wobei der Zylinder (2) eine Innenwand (21), eine Außenwand und einen Auslass an einem Auslassende (23) gegenüber einem Betätigungsende (24) aufweist,
- eine Kolbenanordnung (3), die einen komprimierbaren Abschnitt (31) zwischen einem Betätigungskolbenelement (32) mit einer Shore-A-Härte im Bereich von 50 bis 90 und einem passiven Kolbenelement (33) mit einer Shore-A-Härte im Bereich von 50 bis 90 umfasst, wobei die Kolbenelemente (32, 33) an aneinanderstoßenden Grenzflächen an die Innenwand (21) des Zylinders (2) anstoßen, wodurch der komprimierbare Abschnitt (31) abgedichtet wird, wenn die Kolbenanordnung (3) im Zylinder (2) eingeführt ist, **dadurch gekennzeichnet, dass** der komprimierbare Abschnitt (31) ein komprimierbares Fluid und einen federnden Rahmen (34) umfasst, wobei der federnde Rahmen (34) das Betätigungskolbenelement (32) physisch mit dem passiven Kolbenelement (33) koppelt, wobei der federnde Rahmen (34) aus einem elastischen Polymer wie beispielsweise einem thermoplastischen Elastomer (TPE) mit einer Shore-A-Härte im Bereich von 50 bis 90 hergestellt ist und einen Volumenanteil im Bereich von 15% bis 50% des Volumens des komprimierbaren Abschnitts (31) aufweist, und wobei der federnde Rahmen (34) eine axiale Länge im Bereich von 25% bis 90% der Gesamtaxiallänge der Kolbenanordnung (3) in einem entspannten Zustand aufweist, und dass das komprimierbare Fluid zwischen dem Betätigungskolbenelement (32) und dem passiven Kolbenelement (33) eingeschlossen ist.

2. Injektor (1) nach Anspruch 1, wobei die Losreißkraft (BLF) weniger als doppelt so hoch wie die Gleitkraft ist.

3. Injektor (1) nach einem der Ansprüche 1 oder 2, wobei der Durchmesser des Betätigungskolbenelements kleiner als der Durchmesser des passiven Kolbenelements ist, wenn sich die Kolbenanordnung in einem entspannten Zustand befindet.

4. Injektor (1) nach einem der Ansprüche 1 bis 3, wobei das Betätigungskolbenelement (32), das passive Kolbenelement (33) oder der federnde Rahmen (34) oder das Betätigungskolbenelement (32), das passive Kolbenelement (33) und der federnde Rahmen (34) aus einem thermoplastischen Elastomer (TPE) hergestellt sind.

5. Injektor (1) nach Anspruch 1, wobei das TPE einen Druckverformungsrestwert von mindestens 25% bei einer Temperatur im Bereich von 10°C bis 40°C über eine Zeitdauer im Bereich von 18 Stunden bis 96 Stunden gemäß dem ISO-Standard 815 aufweist.

6. Injektor (1) nach einem der Ansprüche 1 bis 5, wobei der Zylinder (2) einen Innendurchmesser aufweist und wobei das Betätigungskolbenelement (32) und das passive Kolbenelement (33) jeweils ein konvexes verformbares Dichtungselement mit einem Durchmesser von 3% bis 20% größer als der Innendurchmesser des Zylinders (2) umfassen.

7. Injektor (1) nach einem der Ansprüche 1 bis 6, wobei das Betätigungskolbenelement (32), das passive Kolbenelement (33) und der federnde Rahmen (34) als ein einziges Teil spritzgegossen sind.

8. Injektor (1) nach einem der Ansprüche 1 bis 7, wobei die Kolbenanordnung (3) bezüglich einer Betätigungsfläche (321) des Betätigungskolbenelements (32) verglichen mit einer Auslassfläche (331) des passiven Kolbenelements (33) symmetrisch ist.

9. Injektor (1) nach einem der Ansprüche 1 bis 8, wobei der Injektor (1) eine Kolbenstange (6) umfasst und das Betätigungskolbenelement (32) eine Eingriffsvorrichtung (61) zur Ineingriffnahme einer komplementären Eingriffsvorrichtung (62) der Kolbenstange (6) umfasst.

10. Injektor (1) nach Anspruch 8, wobei das Betätigungskolbenelement (32) und das passive Kolbenelement (33) keine Eingriffsvorrichtung (61) zur Ineingriffnahme einer komplementären Eingriffsvorrichtung (62) einer Kolbenstange (6) umfassen.

11. Injektor (1) nach einem der Ansprüche 1 bis 10, wobei der Zylinder (2) aus Glas, einem polymeren Material, einem Metall oder einer beliebigen Kombination von polymeren Materialien, Gläsern oder Metallen hergestellt ist.

12. Injektor (1) nach einem der Ansprüche 1 bis 11, wobei das Betätigungskolbenelement (32), das passive Kolbenelement (33) und/oder der federnde Rahmen (34) eine oder mehrere Stabilisierungsrippen (71) umfassen.

13. Injektor (1) nach einem der Ansprüche 1 bis 12, wobei der Injektor (1) eine Nadelkappe umfasst, die einen röhrenförmigen Abschnitt zum Betätigen des Betätigungskolbenelements (32) aufweist, wobei die Nadelkappe ein Nadeleinführungsende aufweist, das eine Eingriffsvorrichtung zur Ineingriffnahme einer komplementären Eingriffsvorrichtung des Auslasses des Zylinders (2), wenn die Nadelkappe auf dem Zylinder (2) angebracht ist, umfasst, wobei die Nadelkappe eine Länge aufweist, die gleich einer oder größer als eine Betriebslänge des Zylinders (2) ist, die durch den Abstand von dem Betätigungsende (24) des Zylinders (2) zu dem Auslassende (23) des Zylinders (2) minus der Abmessung der Kolbenanordnung (3) parallel zur Längsachse definiert wird.

14. Injektor (1) nach einem der Ansprüche 1 bis 13, wobei der Zylinder (2) einen Innendurchmesser im Bereich von 2 mm bis 10 mm aufweist.

15. Injektor (1) nach einem der Ansprüche 1 bis 14, wobei der Zylinder (2) mit einer pharmazeutischen Zusammensetzung vorgefüllt ist.

## Revendications

1. Injecteur (1) pour l'administration d'une composition pharmaceutique, lequel injecteur (1) ne comprend pas de lubrifiant siliconé, l'injecteur (1) comprenant optionnellement une tige de piston, et lequel injecteur (1) comprend
- un cylindre (2) s'étendant le long d'un axe longitudinal, le cylindre (2) ayant une paroi intérieure (21), une paroi extérieure, et une sortie à une extrémité de sortie (23) en face d'une extrémité d'actionnement (24),
- un ensemble piston (3) comprenant une section compressible (31) entre un élément piston d'actionnement (32) ayant une dureté Shore A dans la plage de 50 à 90 et un élément piston passif (33) ayant une dureté Shore A dans la plage de 50 à 90, lesquels éléments pistons (32, 33) sont contigus à la paroi intérieure (21) du cylindre (2) au niveau d'interfaces de contiguïté, ainsi rendant étanche la section compressible (31) lorsque l'ensemble piston (3) est inséré dans le cylindre (2), **caractérisé en ce que** la section compressible (31) comprend un fluide compressible et un cadre résilient (34), le cadre résilient (34) couplant physiquement l'élément piston d'actionnement (32) à l'élément piston passif (33), lequel cadre résilient (34) est fait d'un polymère élastique, tel qu'un élastomère thermoplastique (TPE) ayant une dureté Shore A dans la plage de 50 à 90, et a une fraction volumétrique dans la plage de 15 % à 50 % du volume de la section compressible (31) et lequel cadre résilient (34) a une longueur axiale dans la plage de 25 % à 90 % de la longueur axiale totale de l'ensemble piston (3) dans un état relâché, et **en ce que** le fluide compressible est piégé entre l'élément piston d'actionnement (32) et l'élément piston passif (33).

2. Injecteur (1) selon la revendication 1, dans lequel la force de libération (BLF) est moins de deux fois plus élevée que la force de glissement.

3. Injecteur (1) selon l'une quelconque des revendications 1 ou 2, dans lequel le diamètre de l'élément piston d'actionnement est inférieur au diamètre de l'élément piston passif lorsque l'ensemble piston est dans un état relâché.

4. Injecteur (1) selon l'une quelconque des revendications 1 à 3, dans lequel l'élément piston d'actionnement (32), l'élément piston passif (33), ou le cadre résilient (34), ou l'élément piston d'actionnement (32), l'élément piston passif (33) et le cadre résilient (34) sont faits d'un élastomère thermoplastique (TPE).

5. Injecteur (1) selon la revendication 1, dans lequel le TPE a une valeur de consigne de compression d'au moins 25 % à une température dans la plage de 10°C à 40°C durant une période dans la plage de 18 heures à 96 heures selon la norme ISO 815.

6. Injecteur (1) selon l'une quelconque des revendications 1 à 5, dans lequel le cylindre (2) a un diamètre intérieur, et l'élément piston d'actionnement (32) et l'élément piston passif (33) comprennent chacun un élément d'étanchéité déformable convexe ayant un diamètre supérieur de 3 % à 20 % au diamètre intérieur du cylindre (2).

7. Injecteur (1) selon l'une quelconque des revendications 1 à 6, dans lequel l'élément piston d'actionnement (32), l'élément piston passif (33) et le cadre résilient (34) sont moulés par injection sous forme de pièce unique.

8. Injecteur (1) selon l'une quelconque des revendications 1 à 7, dans lequel l'ensemble piston (3) est symétrique en ce qui concerne une surface d'actionnement (321) de l'élément piston d'actionnement (32) par rapport à une surface de sortie (331) de l'élément piston passif (33).

9. Injecteur (1) selon l'une quelconque des revendications 1 à 8, dans lequel l'injecteur (1) comprend une tige de piston (6), et l'élément piston d'actionnement (32) comprend un dispositif d'entrée en prise (61) destiné à entrer en prise avec un dispositif d'entrée en prise complémentaire (62) de la tige de piston (6).

10. Injecteur (1) selon la revendication 8, dans lequel l'élément piston d'actionnement (32) et l'élément piston passif (33) ne comprennent pas de dispositif d'entrée en prise (61) destiné à entrer en prise avec un dispositif d'entrée en prise complémentaire (62) d'une tige de piston (6).

11. Injecteur (1) selon l'une quelconque des revendications 1 à 10, dans lequel le cylindre (2) est fait de verre, d'un matériau polymérique, d'un métal, ou d'une quelconque association de matériaux polymériques, de verres ou de métaux.

12. Injecteur (1) selon l'une quelconque des revendications 1 à 11, dans lequel l'élément piston d'actionnement (32), l'élément piston passif (33) et/ou le cadre résilient (34) comprennent une ou plusieurs ailettes stabilisatrices (71).

13. Injecteur (1) selon l'une quelconque des revendications 1 à 12, dans lequel l'injecteur (1) comprend un capuchon d'aiguille ayant une section tubulaire pour actionner l'élément piston d'actionnement (32), lequel capuchon d'aiguille a une extrémité d'insertion d'aiguille comprenant un dispositif d'entrée en prise destiné à entrer en prise avec un dispositif d'entrée en prise complémentaire de la sortie du cylindre (2) lorsque le capuchon d'aiguille est monté sur le cylindre (2), lequel capuchon d'aiguille a une longueur qui est égale ou supérieure à une longueur de fonctionnement du cylindre (2) définie par la distance depuis l'extrémité d'actionnement (24) du cylindre (2) jusqu'à l'extrémité de sortie (23) du cylindre (2) moins la dimension de l'ensemble piston (3) parallèle à l'axe longitudinal.

14. Injecteur (1) selon l'une quelconque des revendications 1 à 13, dans lequel le cylindre (2) a un diamètre intérieur dans la plage de 2 mm à 10 mm.

15. Injecteur (1) selon l'une quelconque des revendications 1 à 14, dans lequel le cylindre (2) est pré-rempli avec une composition pharmaceutique.
